(19) Europäisches Patentamt European Patent Office Office européen des brevets

(11) **EP 4 071 763 A1**

(12) **EUROPEAN PATENT APPLICATION**

(43) Date of publication:
**12.10.2022 Bulletin 2022/41**

(21) Application number: **21167494.0**

(22) Date of filing: **08.04.2021**

(51) International Patent Classification (IPC):
***G16B 40/20*** (2019.01)

(52) Cooperative Patent Classification (CPC):
**G16B 40/20; G01N 33/04; G16B 40/10;**
G01N 21/3577; G01N 2021/3595; G01N 2201/129;
G01N 2201/1296

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **DuPont Nutrition Biosciences ApS
1411 Køpenhaven K (DK)**

(72) Inventors:
• **KJÆR, Karina Hansen
DK-1411 Copenhagen K (DK)**

• **JENSEN, Henrik Max
DK-1411 Copenhagen K (DK)**
• **EWERT, Jacob
DK-1411 Copenhagen K (DK)**
• **CRAMER, Jacob
DK-1411 Copenhagen K (DK)**
• **STEPHANSEN, Julie
DK-1411 Copenhagen K (DK)**

(74) Representative: **D Young & Co LLP
120 Holborn
London EC1N 2DY (GB)**

(54) **A METHOD FOR MEASURING GALACTOOLIGOSACCHARIDES**

(57)    The present invention relates to an in-line method of galactooligosaccharide (GOS) quantification while preparing a dairy product having a high content of GOS fiber, and/or a GOS fiber-enriched dairy product in which the lactose content has also been significantly reduced.

EP 4 071 763 A1

**Description**

**FIELD OF THE INVENTION**

The present invention relates to an in-line method of galactooligosaccharide (GOS) quantification while preparing a dairy product having a high content of GOS fiber, and/or a GOS fiber-enriched dairy product in which the lactose content has also been significantly reduced.

**BACKGROUND OF THE INVENTION**

Galactooligosaccharides (GOS) are carbohydrates defined as having two or more galactose moieties, including as many as nine, linked by glycosidic bonds. Humans and animals are unable to digest GOS. GOS may also include one or more non-galactose sugar moieties, including glucose. One of the beneficial effects of GOS ingestion is its ability to act as prebiotic by selectively stimulating the proliferation of beneficial colonic microorganisms such as bacteria to give physiological benefits. The established health effects have resulted in a growing interest in GOS as a food.

The enzyme β-galactosidase (EC 3.2.1.23) can catalyze two types of reactions. For most β-galactosidases, the hydrolyses of lactose to the monosaccharides D-glucose and D-galactose is the preferred reaction. During catalysis of that reaction, the enzyme hydrolyses lactose and transiently binds the galactose monosaccharide in a galactose-enzyme complex that transfers galactose to the hydroxyl group of water, resulting in the liberation of D-galactose and D-glucose. However, under certain conditions such as high lactose concentrations and/or high temperature, β-galactosidases can transfer galactose to the hydroxyl groups of D-galactose or D-glucose. This reaction is called transgalactosylation. The main product of transgalactosylation is GOS.

Enzymes and methods for creating high levels of GOS have been developed. See, e.g., Polypeptides Having Transgalactosylating Activity, WO 2013/182686. Additionally, methods for creating high levels of GOS while also further reducing the remaining lactose have been developed. See, e.g., Use of lactase to provide high GOS fiber level and low lactose level, WO 2020/117548. In the context of dairy applications for milk-based products such as yogurt, cheese, milk beverages and milk powders, while production of GOS reduces the endogenous lactose sugar, lactose levels may remain too high for individuals with lactose intolerance. It is estimated that some 70 % of the world's population are lactose intolerant, i.e. suffer from digestive disorders if they consume lactose.

As noted above, certain β-galactosidases under the correct conditions can drive the transformation of lactose into GOS. However, these same enzymes can catalyze the hydrolysis of GOS into glucose and galactose (depending on the composition of the GOS) under the correct conditions. Typically, this happens at or near peak GOS formation. GOS hydrolysis can be prevented by enzyme inactivation (for example by heat). However, knowing when to inactivate requires reliable and accurate information as to the extent of transgalactosylation and, in particular, when the optimal GOS concentration has been reached.

There is a continuing need in the art for methods to determine optimal GOS yields. This is particularly true in industrial dairy settings where milk producers are manipulating hundreds of thousands of liters of milk in a short amount of time and do not have sophisticated laboratory equipment or trained chemists on premises.

**SUMMARY OF THE INVENTION**

A method is presented for determining carbohydrate content in a sample, the method having the steps of: obtaining FTIR (Fourier Transform Infrared Spectroscopy) spectrum data corresponding to the sample; providing at least a portion of the FTIR spectrum data as an input to a trained machine learning model; and processing the at least a portion of the FTIR spectrum data using the trained machine learning model to generate a carbohydrate content value providing a quantitative indication of a level of carbohydrate content in the sample.

Optionally, the carbohydrate is one or more of GOS, DP3+ GOS, glucose, galactose, and DP2. Optionally, DP2 is lactose. Optionally, the carbohydrate is DP3+ GOS. Optionally, the sample is a milk-based substrate. Optionally, the portion of the FTIR spectrum data supplied as the input to the trained machine learning model is FTIR spectrum data within a limited spectral range. Optionally, the limited spectral range includes $1046 cm^{-1}$, $1076 cm^{-1}$, $1157 cm^{-1}$ and $1250 cm^{-1}$. Optionally, the limited spectral range is a wavenumber region for which a lower bound is between $900 cm^{-1}$ and $1100 cm^{-1}$ and an upper bound is between $1300 cm^{-1}$ and $1500 cm^{-1}$. Optionally, the limited spectral range is a wavenumber region for which a lower bound is between $1008 cm^{-1}$ and $1068 cm^{-1}$ and an upper bound is between $1414 cm^{-1}$ and $1475 cm^{-1}$. Optionally, the limited spectral range is in wavenumber region $1037{:}1450 cm^{-1}$.

Optionally, the trained machine learning model is a supervised learning model trained using a training data set having, for each of a plurality of training samples, the FTIR spectrum data corresponding to the training sample and a measured indication of the level of carbohydrate content in the training sample.

Optionally, the trained machine learning model is a partial least squares regression (PLSR) model.

Optionally, the trained machine learning model is Neural Network regression model.

Optionally, the trained machine learning model comprises multiple-linear regression (MLR).

Optionally, the trained machine learning model is principle components regression (PCR).

Optionally, the trained machine learning model comprises classical least squares method CLS.

Optionally, the FTIR spectrum data is obtained from a server-based data store to which the FTIR spectrum data is uploaded by a client device.

Optionally, the processing of at least a portion of the FTIR spectrum data using the trained machine learning model is performed at a server device using the FTIR spectrum data obtained from a client device.

Optionally, the carbohydrate content value is made accessible to the client device.

A method is presented for training a machine learning model to predict carbohydrate content in a milk-based substrate; the method having the steps of: obtaining a training data set having, for each of a plurality of training samples, FTIR (Fourier Transform Infrared Spectroscopy) spectrum data corresponding to the training sample and a measured indication of a level of carbohydrate content in the training sample; and performing supervised learning using the training data set, to determine trained model coefficients for the machine learning model.

Optionally, the carbohydrate is one or more of GOS, DP3+ GOS, glucose, galactose, and DP2. Optionally, the DP2 is lactose. Optionally, the carbohydrate is DP3+ GOS.

In another aspect of the invention, a computer program is presented which, when executed on a data processing apparatus, controls the data processing apparatus to perform the method as described above.

In another aspect of the present invention, a method for preparing a milk product containing carbohydrate is presented, having the steps of: treating a milk-based substrate with a trans-galactosylating enzyme; performing FTIR (Fourier Transform Infrared Spectroscopy) on a sample of the milk-based substrate to obtain FTIR spectrum data corresponding to the sample; obtaining, based on processing of at least a portion of the FTIR spectrum data using a trained machine learning model, a carbohydrate content value providing a quantitative indication of a level of carbohydrate content in the sample; and determining, based on the carbohydrate content value, when to inactivate the trans-galactosylating enzyme by pasteurization of the milk base.

Optionally, the carbohydrate is one or more of GOS, DP3+ GOS, glucose, galactose, and DP2. Optionally, the DP2 is lactose. Optionally, the carbohydrate is DP3+ GOS. 29.

Optionally, at least a portion of the FTIR spectrum data is uploaded by a client device to a server for server-based processing by the trained machine learning model to generate the carbohydrate content value, and the carbohydrate content value is obtained by the client device as a result of the server-based processing.

Optionally, the method for preparing a milk based carbohydrate has an accuracy better than 10%, expressed as Standard Error of Prediction at mean value (3.75 %), the concentration of GOS in a concentration range of 0-7.5 % in a milk base containing at least 0.1% fat, at least 0.5% dissolved lactose, and at least 1% protein.

Optionally, the method for preparing a milk based carbohydrate has a linearity ($R^2$) of the PLS regression model above 0.9 (less preferred 0.85, 0.8, 0.75 and so forth) to validate the GOS content.

Optionally, the trans-galactosylating enzyme is derived from *Bifidobacterium bifidum.* Optionally, the transgalactosylating enzyme is a truncated β-galactosidase from *Bifidobacterium bifidum.* Optionally, the truncated β-galactosidase from *Bifidobacterium bifidum* is truncated on the C-terminus. Optionally, the truncated β-galactosidase from *Bifidobacterium bifidum* is a polypeptide having at least 70% sequence identity to SEQ ID. NO:1, SEQ ID NO:2, SEQ ID NO:3, SEQ ID NO:4 or SEQ ID NO:5 or to a transgalactosylase active fragment thereof.

Optionally, the polypeptide has at least 80% sequence identity to SEQ ID. NO:1, SEQ ID NO:2, SEQ ID NO:3, SEQ ID NO:4 or SEQ ID NO:5 or to a transgalactosylase active fragment thereof. Optionally, the polypeptide has at least 90% sequence identity to SEQ ID. NO:1, SEQ ID NO:2, SEQ ID NO:3, SEQ ID NO:4 or SEQ ID NO:5 or to a transgalactosylase active fragment thereof. Optionally, the polypeptide has at least 95% sequence identity to SEQ ID. NO:1, SEQ ID NO:2, SEQ ID NO:3, SEQ ID NO:4 or SEQ ID NO:5 or to a transgalactosylase active fragment thereof. Optionally, the polypeptide has at least 99% sequence identity to SEQ ID. NO:1, SEQ ID NO:2, SEQ ID NO:3, SEQ ID NO:4 or SEQ ID NO:5 or to a transgalactosylase active fragment thereof. Optionally, the polypeptide is a sequence according to SEQ ID. NO:1, SEQ ID NO:2, SEQ ID NO:3, SEQ ID NO:4 or SEQ ID NO:5 or to a transgalactosylase active fragment thereof. Optionally, the polypeptide is a sequence according to SEQ ID NO:1

## BRIEF DESCRIPTION OF THE DRAWINGS

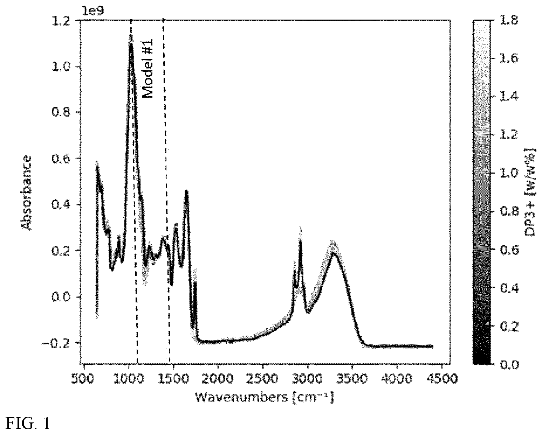

Figure 1: The absorbance mid-infrared spectra from 10 multiple scatter corrected spectra of dried milk samples containing GOS analyzed by Perkin Elmer Spectrum One FTIR instrument in the wavenumber-range 650-4400 cm$^{-1}$ using a spectral resolution of 4cm$^{-1}$. The black/white bar is shaded according to the GOS (DP3+) level as measured by HPLC (example 2) as shown on second axis in w/w %. Wavenumber regions: #1 (1037 -1445 cm$^{-1}$), #2 (650-4400

cm$^{-1}$) and #3 (650-1036 + 1449-4400 cm$^{-1}$) are marked in spectrum with lines (used for modelling in example 10). The optimal model (Model #1) is marked with two dotted lines.

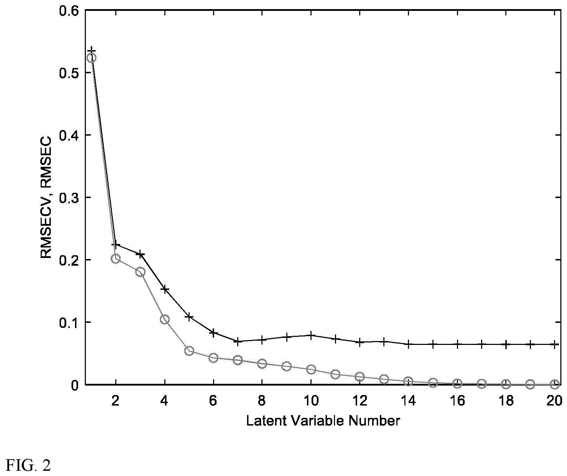

Figure 2. The prediction errors (RMSECV (+), RMSC (o)) plotted against number of PLS components/Latent Variables used in the Model #1.

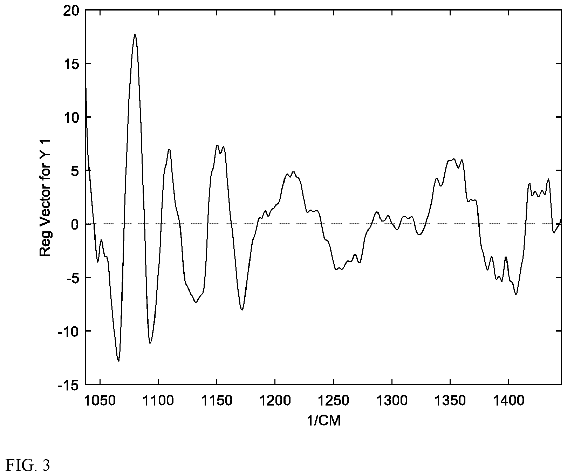

Figure 3: Model coefficients: the regression vector of the prediction of DP3+ (Y1) from Model#1 as a function of wavenumber (cm-1).

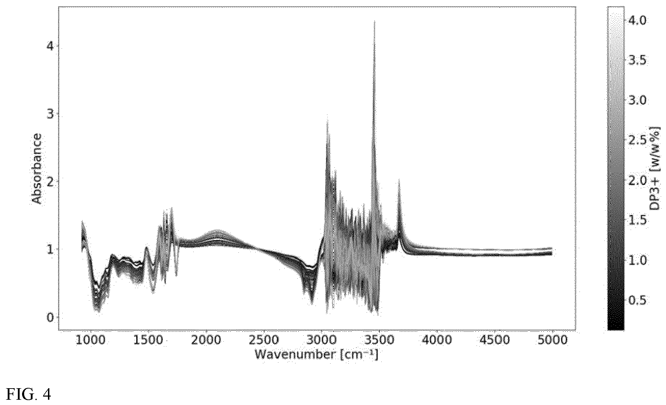

Figure 4: The spectral data from 476 (119x4 replicates) GOS containing milk samples analyzed by the MilkoScan FT2 instrument, represented by mid-infrared absorbance as function of wavenumber. The color bar is colored according to the GOS (% (w/w) DP3+) level as measured by HPLC as shown on second axis in %.

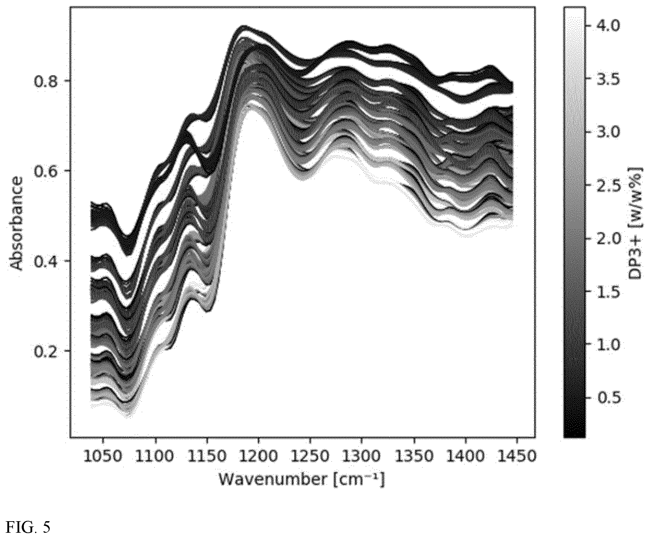

Figure 5: Zoomed region (wavenumber range: 1038 cm$^{-1}$-1446 cm$^{-1}$ incl.) of spectral data from 476 (119x4 replicates) of GOS (DP3+) containing milk samples analyzed by the MilkoScan FT2 instrument, represented by absolute mid-infrared absorbance as function of wavenumber (The color bar is colored according to the GOS level as measured by HPLC as shown on second axis in %.

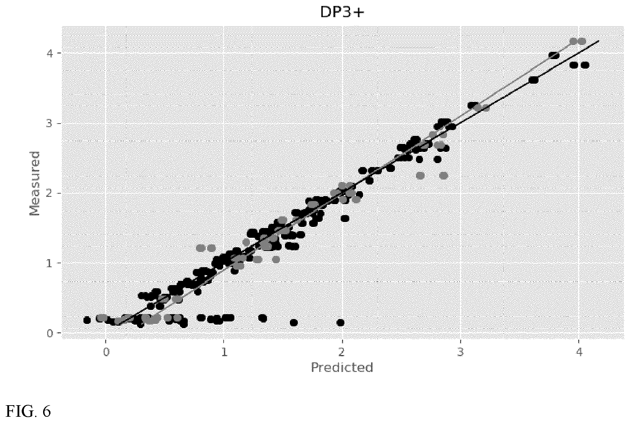

Figure 6: Model prediction of DP3+ in w/w % vs measured DP3+ in w/w %. Data from the training set is colored black, and data from the test set gray. The gray line illustrates a least square regression fit of a straight line to measured vs predicted values, while the gray line illustrates the perfect model having predicted values equal to measured values.

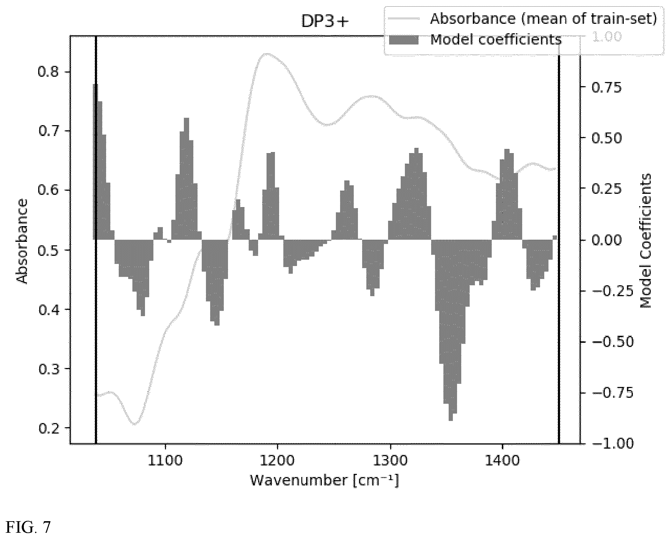

Figure 7: The mean absorbance spectra of the training dataset within the range of Model#1 (left hand side axis), along with the model coefficients of Model#1 (right hand side axis).

## DETAILED DISCLOSURE OF THE INVENTION

Detailed description of the invention:

The practice of the present teachings will employ, unless otherwise indicated, conventional techniques of molecular biology (including recombinant techniques), microbiology, cell biology, and biochemistry, which are within the skill of the art. Such techniques are explained fully in the literature, for example, Molecular Cloning: A Laboratory Manual, second edition (Sambrook et al., 1989); Oligonucleotide Synthesis (M. J. Gait, ed., 1984; Current Protocols in Molecular Biology (F. M. Ausubel et al., eds., 1994); PCR: The Polymerase Chain Reaction (Mullis et al., eds., 1994); Gene Transfer and Expression: A Laboratory Manual (Kriegler, 1990), and The Alcohol Textbook (Ingledew et al., eds., Fifth Edition, 2009), and Essentials of Carbohydrate Chemistry and Biochemistry (Lindhorste, 2007).
Unless defined otherwise herein, all technical and scientific terms used herein have the same meaning as commonly understood by one of ordinary skill in the art to which the present teachings belong. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present teachings.
Numeric ranges provided herein are inclusive of the numbers defining the range.

Definitions:

A "β-galactosidase" is glycoside hydrolase that catalyzes the hydrolysis of β-galactosidase, including lactose, into monosaccharides. A β-galactosidase is also sometimes called a "lactase."
The term "galactooligosaccharide" also referred to herein as "GOS" refers to nondigestable oligosaccharides composed of from 2 to 20 molecules of predominantly galactose. GOS is typically formed by β-galactosidase enzymes, also called lactases by degrading lactose in e.g. milk and/or milk-based products.
The term "GOS fiber" which is equivalent to "DP3+ GOS" herein refers to nondigestable galactooligosaccharides with a degree of polymerization of 3 or more molecules of predominantly galactose.
DP2 herein refers to species of disaccharides, including lactose, GOS disaccharides (e.g. allolactose).
The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleotide change. However, note that a polynucleotide encoding a wild-type, parental, or reference polypeptide is not limited to a naturally-occurring polynucleotide, and encompasses any polynucleotide encoding the wild-type, parental, or reference polypeptide.

Reference to the wild-type polypeptide is understood to include the mature form of the polypeptide. A "mature" polypeptide or variant, thereof, is one in which a signal sequence is absent, for example, cleaved from an immature form of the polypeptide during or following expression of the polypeptide.

The term "variant," with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

The term "recombinant," when used in reference to a subject cell, nucleic acid, protein or vector, indicates that the subject has been modified from its native state. Thus, for example, recombinant cells express genes that are not found within the native (non-recombinant) form of the cell, or express native genes at different levels or under different conditions than found in nature. Recombinant nucleic acids differ from a native sequence by one or more nucleotides and/or are operably linked to heterologous sequences, e.g., a heterologous promoter in an expression vector. Recombinant proteins may differ from a native sequence by one or more amino acids and/or are fused with heterologous sequences. A vector comprising a nucleic acid encoding a β-galactosidase is a recombinant vector.

The terms "recovered," "isolated," and "separated," refer to a compound, protein (polypeptides), cell, nucleic acid, amino acid, or other specified material or component that is removed from at least one other material or component with which it is naturally associated as found in nature. An "isolated" polypeptides, thereof, includes, but is not limited to, a culture broth containing secreted polypeptide expressed in a heterologous host cell.

The term "polymer" refers to a series of monomer groups linked together. A polymer is composed of multiple units of a single monomer. As used herein the term "glucose polymer" refers to glucose units linked together as a polymer. As long as there are at least three glucose units, the glucose polymer may contain non-glucose sugars such as lactose or galactose.

The term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (i.e., N→C).

The term "nucleic acid" encompasses DNA, RNA, heteroduplexes, and synthetic molecules capable of encoding a polypeptide. Nucleic acids may be single stranded or double stranded and may be chemically modified. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

The terms "transformed," "stably transformed," and "transgenic," used with reference to a cell means that the cell contains a non-native (e.g., heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest (e.g., a β-galactosidase) has been introduced. Exemplary host strains are microorganism cells (e.g., bacteria, filamentous fungi, and yeast) capable of expressing the polypeptide of interest. The term "host cell" includes protoplasts created from cells.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

The term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (e.g., hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequences.

A "signal sequence" is a sequence of amino acids attached to the N-terminal portion of a protein, which facilitates the secretion of the protein outside the cell. The mature form of an extracellular protein lacks the signal sequence, which is cleaved off during the secretion process.

"Biologically active" refers to a sequence having a specified biological activity, such an enzymatic activity.

The term "specific activity" refers to the number of moles of substrate that can be converted to product by an enzyme or enzyme preparation per unit time under specific conditions. Specific activity is generally expressed as units (U)/mg of protein.

As used herein, "percent sequence identity" means that a particular sequence has at least a certain percentage of amino acid residues identical to those in a specified reference sequence, when aligned using the CLUSTAL W algorithm with default parameters. *See* Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

Deletions are counted as non-identical residues, compared to a reference sequence. Deletions occurring at either terminus are included. For example, a variant with five amino acid deletions of the C-terminus of the mature 617 residue polypeptide would have a percent sequence identity of 99% (612/617 identical residues $\times$ 100, rounded to the nearest whole number) relative to the mature polypeptide. Such a variant would be encompassed by a variant having "at least 99% sequence identity" to a mature polypeptide.

"Fused" polypeptide sequences are connected, *i.e.*, operably linked, via a peptide bond between two subject polypeptide sequences.

The term "filamentous fungi" refers to all filamentous forms of the subdivision Eumycotina, particularly Pezizomycotina species.

The term "about" refers to $\pm$ 5% to the referenced value.

"Lactase treated milk" means milk treated with lactase to reduce the amount of lactose sugar.

"Reduced lactose milk" means milk wherein the percentage of lactose is about 2% or lower.

"Lactose free milk" means milk wherein the percentage of lactose is below 0.1% (w/v) and in some instances this would mean milk wherein the percentage of lactose is below 0.01% (w/v).

The term "milk", in the context of the present invention, is to be understood as the lacteal secretion obtained from any mammal, such as cows, sheep, goats, buffaloes or camels.

In the present context, the term "milk-based substrate" means any raw and/or processed milk material or a material derived from milk constituents. Useful milk-based substrates include but are not limited to solutions/suspensions of any milk or milk like products comprising lactose, such as whole or low fat milk, skim milk, buttermilk, reconstituted milk powder, condensed milk, solutions of dried milk, UHT milk, whey, whey permeate, acid whey, or cream. Preferably, the milk-based substrate is milk or an aqueous solution of skim milk powder. The milk-based substrate may be more concentrated than raw milk. In one embodiment, the milk-based substrate has a ratio of protein to lactose of at least 0.2, preferably at least 0.3, at least 0.4, at least 0.5, at least 0.6 or, most preferably, at least 0.7. The milk-based substrate may be homogenized and/or pasteurized according to methods known in the art.

**Sequences**

The amino acid sequence of the mature truncated form of β-galactosidase from *Bifidobacterium bifidum,* BIF917, is set forth as SEQ ID NO:1:

VEDATRSDSTTQMSSTPEVVYSSAVDSKQNRTSDFDANWKFMLSDSVQAQDPAFDD

SAWQQVDLPHDYSITQKYSQSNEAESAYLPGGTGWYRKSFTIDRDLAGKRIAINFDG

VYMNATVWFNGVKLGTHPYGYSPFSFDLTGNAKFGGENTIVVKVENRLPSSRWYSG

SGIYRDVTLTVTDGVHVGNNGVAIKTPSLATQNGGDVTMNLTTKVANDTEAAANIT

LKQTVFPKGGKTDAAIGTVTTASKSIAAGASADVTSTITAASPKLWSIKNPNLYTVRT

EVLNGGKVLDTYDTEYGFRWTGFDATSGFSLNGEKVKLKGVSMHHDQGSLGAVAN

RRAIERQVEILQKMGVNSIRTTHNPAAKALIDVCNEKGVLVVEEVFDMWNRSKNGN

TEDYGKWFGQAIAGDNAVLGGDKDETWAKFDLTSTINRDRNAPSVIMWSLGNEMM

EGISGSVSGFPATSAKLVAWTKAADSTRPMTYGDNKIKANWNESNTMGDNLTANG

GVVGTNYSDGANYDKIRTTHPSWAIYGSETASAINSRGIYNRTTGGAQSSDKQLTSY

DNSAVGWGAVASSAWYDVVQRDFVAGTYVWTGFDYLGEPTPWNGTGSGAVGSW

PSPKNSYFGIVDTAGFPKDTYYFQSQWNDDVHTLHILPAWNENVVAKGSGNNVPV

VVYTDAAKVKLYFTPKGSTEKRLIGEKSFTKKTTAAGYTYQVYEGSDKDSTAHKNM

YLTWNVPWAEGTISAEAYDENNRLIPEGSTEGNASVTTTGKAAKLKADADRKTITA

DGKDLSYIEVDVTDANGHIVPDAANRVTFDVKGAGKLVGVDNGSSPDHDSYQADN

RKAFSGKVLAIVQSTKEAGEITVTAKADGLQSSTVKIATTAVPGTSTEKT

The amino acid sequence of the mature truncated form of β-galactosidase from *Bifidobacterium bifidum,* BIF995, is set forth as SEQ ID NO:2:

VEDATRSDSTTQMSSTPEVVYSSAVDSKQNRTSDFDANWKFMLSDSVQAQDPAFDD SAWQQVDLPHDYSITQKYSQSNEAESAYLPGGTGWYRKSFTIDRDLAGKRIAINFDG VYMNATVWFNGVKLGTHPYGYSPFSFDLTGNAKFGGENTIVVKVENRLPSSRWYSG SGIYRDVTLTVTDGVHVGNNGVAIKTPSLATQNGGDVTMNLTTKVANDTEAAANIT LKQTVFPKGGKTDAAIGTVTTASKSIAAGASADVTSTITAASPKLWSIKNPNLYTVRT EVLNGGKVLDTYDTEYGFRWTGFDATSGFSLNGEKVKLKGVSMHHDQGSLGAVAN RRAIERQVEILQKMGVNSIRTTHNPAAKALIDVCNEKGVLVVEEVFDMWNRSKNGN TEDYGKWFGQAIAGDNAVLGGDKDETWAKFDLTSTINRDRNAPSVIMWSLGNEMM EGISGSVSGFPATSAKLVAWTKAADSTRPMTYGDNKIKANWNESNTMGDNLTANG GVVGTNYSDGANYDKIRTTHPSWAIYGSETASAINSRGIYNRTTGGAQSSDKQLTSY DNSAVGWGAVASSAWYDVVQRDFVAGTYVWTGFDYLGEPTPWNGTGSGAVGSW PSPKNSYFGIVDTAGFPKDTYYFYQSQWNDDVHTLHILPAWNENVVAKGSGNNVPV VVYTDAAKVKLYFTPKGSTEKRLIGEKSFTKKTTAAGYTYQVYEGSDKDSTAHKNM YLTWNVPWAEGTISAEAYDENNRLIPEGSTEGNASVTTTGKAAKLKADADRKTITA DGKDLSYIEVDVTDANGHIVPDAANRVTFDVKGAGKLVGVDNGSSPDHDSYQADN RKAFSGKVLAIVQSTKEAGEITVTAKADGLQSSTVKIATTAVPGTSTEKTVRSFYYSR NYYVKTGNKPILPSDVEVRYSDGTSDRQNVTWDAVSDDQIAKAGSFSVAGTVAGQ KISVRVTMIDEIGAL

The amino acid sequence of the mature truncated form of β-galactosidase from *Bifidobacterium bifidum,* BIF1068, is set forth as SEQ ID NO:3:

VEDATRSDSTTQMSSTPEVVYSSAVDSKQNRTSDFDANWKFMLSDSVQAQDPAFDD

SAWQQVDLPHDYSITQKYSQSNEAESAYLPGGTGWYRKSFTIDRDLAGKRIAINFDG

VYMNATVWFNGVKLGTHPYGYSPFSFDLTGNAKFGGENTIVVKVENRLPSSRWYSG

SGIYRDVTLTVTDGVHVGNNGVAIKTPSLATQNGGDVTMNLTTKVANDTEAAANIT

LKQTVFPKGGKTDAAIGTVTTASKSIAAGASADVTSTITAASPKLWSIKNPNLYTVRT

EVLNGGKVLDTYDTEYGFRWTGFDATSGFSLNGEKVKLKGVSMHHDQGSLGAVAN

RRAIERQVEILQKMGVNSIRTTHNPAAKALIDVCNEKGVLVVEEVFDMWNRSKNGN

TEDYGKWFGQAIAGDNAVLGGDKDETWAKFDLTSTINRDRNAPSVIMWSLGNEMM

EGISGSVSGFPATSAKLVAWTKAADSTRPMTYGDNKIKANWNESNTMGDNLTANG

GVVGTNYSDGANYDKIRTTHPSWAIYGSETASAINSRGIYNRTTGGAQSSDKQLTSY

DNSAVGWGAVASSAWYDVVQRDFVAGTYVWTGFDYLGEPTPWNGTGSGAVGSW

PSPKNSYFGIVDTAGFPKDTYYFYQSQWNDDVHTLHILPAWNENVVAKGSGNNVPV

VVYTDAAKVKLYFTPKGSTEKRLIGEKSFTKKTTAAGYTYQVYEGSDKDSTAHKNM

YLTWNVPWAEGTISAEAYDENNRLIPEGSTEGNASVTTTGKAAKLKADADRKTITA

DGKDLSYIEVDVTDANGHIVPDAANRVTFDVKGAGKLVGVDNGSSPDHDSYQADN

RKAFSGKVLAIVQSTKEAGEITVTAKADGLQSSTVKIATTAVPGTSTEKTVRSFYYSR

NYYVKTGNKPILPSDVEVRYSDGTSDRQNVTWDAVSDDQIAKAGSFSVAGTVAGQ

KISVRVTMIDEIGALLNYSASTPVGTPAVLPGSRPAVLPDGTVTSANFAVHWTKPAD

TVYNTAGTVKVPGTATVFGKEFKVTATIRVQ

The amino acid sequence of the mature truncated form of β-galactosidase from *Bifidobacterium bifidum*, BIF1172, is set forth as SEQ ID NO:4:

VEDATRSDSTTQMSSTPEVVYSSAVDSKQNRTSDFDANWKFMLSDSVQAQDPAFDD

SAWQQVDLPHDYSITQKYSQSNEAESAYLPGGTGWYRKSFTIDRDLAGKRIAINFDG

VYMNATVWFNGVKLGTHPYGYSPFSFDLTGNAKFGGENTIVVKVENRLPSSRWYSG

SGIYRDVTLTVTDGVHVGNNGVAIKTPSLATQNGGDVTMNLTTKVANDTEAAANIT

LKQTVFPKGGKTDAAIGTVTTASKSIAAGASADVTSTITAASPKLWSIKNPNLYTVRT

EVLNGGKVLDTYDTEYGFRWTGFDATSGFSLNGEKVKLKGVSMHHDQGSLGAVAN

RRAIERQVEILQKMGVNSIRTTHNPAAKALIDVCNEKGVLVVEEVFDMWNRSKNGN

TEDYGKWFGQAIAGDNAVLGGDKDETWAKFDLTSTINRDRNAPSVIMWSLGNEMM

EGISGSVSGFPATSAKLVAWTKAADSTRPMTYGDNKIKANWNESNTMGDNLTANG

GVVGTNYSDGANYDKIRTTHPSWAIYGSETASAINSRGIYNRTTGGAQSSDKQLTSY

DNSAVGWGAVASSAWYDVVQRDFVAGTYVWTGFDYLGEPTPWNGTGSGAVGSW

PSPKNSYFGIVDTAGFPKDTYYFYQSQWNDDVHTLHILPAWNENVVAKGSGNNVPV

VVYTDAAKVKLYFTPKGSTEKRLIGEKSFTKKTTAAGYTYQVYEGSDKDSTAHKNM

YLTWNVPWAEGTISAEAYDENNRLIPEGSTEGNASVTTTGKAAKLKADADRKTITA

DGKDLSYIEVDVTDANGHIVPDAANRVTFDVKGAGKLVGVDNGSSPDHDSYQADN

RKAFSGKVLAIVQSTKEAGEITVTAKADGLQSSTVKIATTAVPGTSTEKTVRSFYYSR

NYYVKTGNKPILPSDVEVRYSDGTSDRQNVTWDAVSDDQIAKAGSFSVAGTVAGQ

KISVRVTMIDEIGALLNYSASTPVGTPAVLPGSRPAVLPDGTVTSANFAVHWTKPAD

TVYNTAGTVKVPGTATVFGKEFKVTATIRVQRSQVTIGSSVSGNALRLTQNIPADKQ

SDTLDAIKDGSTTVDANTGGGANPSAWTNWAYSKAGHNTAEITFEYATEQQLGQIV

MYFFRDSNAVRFPDAGKTKIQI

The amino acid sequence of the mature truncated form of β-galactosidase from *Bifidobacterium bifidum,* BIF1241, is set forth as SEQ ID NO:5:

VEDATRSDSTTQMSSTPEVVYSSAVDSKQNRTSDFDANWKFMLSDSVQAQDPAFDD

SAWQQVDLPHDYSITQKYSQSNEAESAYLPGGTGWYRKSFTIDRDLAGKRIAINFDG

VYMNATVWFNGVKLGTHPYGYSPFSFDLTGNAKFGGENTIVVKVENRLPSSRWYSG

SGIYRDVTLTVTDGVHVGNNGVAIKTPSLATQNGGDVTMNLTTKVANDTEAAANIT

LKQTVFPKGGKTDAAIGTVTTASKSIAAGASADVTSTITAASPKLWSIKNPNLYTVRT

EVLNGGKVLDTYDTEYGFRWTGFDATSGFSLNGEKVKLKGVSMHHDQGSLGAVAN

RRAIERQVEILQKMGVNSIRTTHNPAAKALIDVCNEKGVLVVEEVFDMWNRSKNGN

TEDYGKWFGQAIAGDNAVLGGDKDETWAKFDLTSTINRDRNAPSVIMWSLGNEMM

EGISGSVSGFPATSAKLVAWTKAADSTRPMTYGDNKIKANWNESNTMGDNLTANG

GVVGTNYSDGANYDKIRTTHPSWAIYGSETASAINSRGIYNRTTGGAQSSDKQLTSY

DNSAVGWGAVASSAWYDVVQRDFVAGTYVWTGFDYLGEPTPWNGTGSGAVGSW

PSPKNSYFGIVDTAGFPKDTYYFYQSQWNDDVHTLHILPAWNENVVAKGSGNNVPV

VVYTDAAKVKLYFTPKGSTEKRLIGEKSFTKKTTAAGYTYQVYEGSDKDSTAHKNM

YLTWNVPWAEGTISAEAYDENNRLIPEGSTEGNASVTTTGKAAKLKADADRKTITA

DGKDLSYIEVDVTDANGHIVPDAANRVTFDVKGAGKLVGVDNGSSPDHDSYQADN

RKAFSGKVLAIVQSTKEAGEITVTAKADGLQSSTVKIATTAVPGTSTEKTVRSFYYSR

NYYVKTGNKPILPSDVEVRYSDGTSDRQNVTWDAVSDDQIAKAGSFSVAGTVAGQ

KISVRVTMIDEIGALLNYSASTPVGTPAVLPGSRPAVLPDGTVTSANFAVHWTKPAD

TVYNTAGTVKVPGTATVFGKEFKVTATIRVQRSQVTIGSSVSGNALRLTQNIPADKQ

SDTLDAIKDGSTTVDANTGGGANPSAWTNWAYSKAGHNTAEITFEYATEQQLGQIV
MYFFRDSNAVRFPDAGKTKIQISADGKNWTDLAATETIAAQESSDRVKPYTYDFAPV
GATFVKVTVTNADTTTPSGVVCAGLTEIELKTAT

**Additional mutations**

In some embodiments, the present β-galactosidases further include one or more mutations that provide a further performance or stability benefit. Exemplary performance benefits include but are not limited to increased thermal stability, increased storage stability, increased solubility, an altered pH profile, increased specific activity, modified substrate specificity, modified substrate binding, modified pH-dependent activity, modified pH-dependent stability, increased oxidative stability, and increased expression. In some cases, the performance benefit is realized at a relatively low temperature. In some cases, the performance benefit is realized at relatively high temperature.

Furthermore, the present β-galactosidases may include any number of conservative amino acid substitutions. Exemplary conservative amino acid substitutions are listed in the following Table.

Conservative amino acid substitutions

| For Amino Acid | Code | Replace with any of |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

The reader will appreciate that some of the above mentioned conservative mutations can be produced by genetic manipulation, while others are produced by introducing synthetic amino acids into a polypeptide by genetic or other means.

The present β-galactosidases may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective β-galactosidase polypeptides. The present β-galactosidase polypeptides may also be truncated to remove the N or C-termini, so long as the resulting polypeptides retain β-galactosidase activity.

The present β-galactosidases may be a "chimeric" or "hybrid" polypeptide, in that it includes at least a portion of a first β-galactosidase polypeptide, and at least a portion of a second β-galactosidase polypeptide. The present β-galactosidases may further include heterologous signal sequence, an epitope to allow tracking or purification, or the like. Exemplary heterologous signal sequences are from *B. licheniformis* amylase (LAT), *B. subtilis* (AmyE or AprE), and *Streptomyces* CelA.

**Production of β-galactosidases**

The present β-galactosidases can be produced in host cells, for example, by secretion or intracellular expression. A cultured cell material (*e.g.*, a whole-cell broth) comprising a β-galactosidase can be obtained following secretion of the β-galactosidase into the cell medium. Optionally, the β-galactosidase can be isolated from the host cells, or even isolated from the cell broth, depending on the desired purity of the final β-galactosidase. A gene encoding a β-galactosidase can be cloned and expressed according to methods well known in the art. Suitable host cells include bacterial, fungal (including yeast and filamentous fungi), and plant cells (including algae). Particularly useful host cells include *Aspergillus niger, Aspergillus oryzae* or *Trichoderma reesei*. Other host cells include bacterial cells, *e.g., Bacillus subtilis* or *B. licheniformis,* as well as *Streptomyces, and E. Coli.*

The host cell further may express a nucleic acid encoding a homologous or heterologous β-galactosidase, *i.e.*, a β-galactosidase that is not the same species as the host cell, or one or more other enzymes. The β-galactosidase may be a variant β-galactosidase. Additionally, the host may express one or more accessory enzymes, proteins, peptides.

**Vectors**

A DNA construct comprising a nucleic acid encoding a β-galactosidase can be constructed to be expressed in a host cell. Because of the well-known degeneracy in the genetic code, variant polynucleotides that encode an identical amino acid sequence can be designed and made with routine skill. It is also well-known in the art to optimize codon use for a particular host cell. Nucleic acids encoding β-galactosidase can be incorporated into a vector. Vectors can be transferred to a host cell using well-known transformation techniques, such as those disclosed below.

The vector may be any vector that can be transformed into and replicated within a host cell. For example, a vector comprising a nucleic acid encoding a β-galactosidase can be transformed and replicated in a bacterial host cell as a means of propagating and amplifying the vector. The vector also may be transformed into an expression host, so that the encoding nucleic acids can be expressed as a functional β-galactosidase. Host cells that serve as expression hosts can include filamentous fungi, for example.

A nucleic acid encoding a β-galactosidase can be operably linked to a suitable promoter, which allows transcription in the host cell. The promoter may be any DNA sequence that shows transcriptional activity in the host cell of choice and may be derived from genes encoding proteins either homologous or heterologous to the host cell. Exemplary promoters for directing the transcription of the DNA sequence encoding a β-galactosidase, especially in a bacterial host, are the promoter of the lac operon of *E. coli,* the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus niger* neutral α-amylase, *A. niger* acid stable α-amylase, *A. niger* glucoamylase, *Rhizomucor miehei* lipase, *A. oryzae* alkaline protease, *A. oryzae* triose phosphate isomerase, or *A. nidulans* acetamidase. When a gene encoding a β-galactosidase is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. *cbhl* is an endogenous, inducible promoter from *T. reesei*. See Liu et al. (2008) "Improved heterologous gene expression in Trichoderma reesei by cellobiohydrolase I gene (cbh1) promoter optimization," Acta Biochim. Biophys. Sin (Shanghai) 40(2): 158-65.

The coding sequence can be operably linked to a signal sequence. The DNA encoding the signal sequence may be the DNA sequence naturally associated with the β-galactosidase gene to be expressed or from a different Genus or species. A signal sequence and a promoter sequence comprising a DNA construct or vector can be introduced into a fungal host cell and can be derived from the same source. For example, the signal sequence is the *cbhl* signal sequence that is operably linked to a *cbhl* promoter.

An expression vector may also comprise a suitable transcription terminator and, in eukaryotes, polyadenylation sequences operably linked to the DNA sequence encoding a variant β-galactosidase. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUB110, pE194, pAMB1, and pIJ702.

The vector may also comprise a selectable marker, *e.g.,* a gene the product of which complements a defect in the isolated host cell, such as the *dal* genes from *B. subtilis* or *B. licheniformis,* or a gene that confers antibiotic resistance such as, *e.g.,* ampicillin, kanamycin, chloramphenicol, or tetracycline resistance. Furthermore, the vector may comprise *Aspergillus* selection markers such as *amdS, argB, niaD* and xxsC, a marker giving rise to hygromycin resistance, or the selection may be accomplished by co-transformation, such as known in the art. *See e.g.,* International PCT Application WO 91/17243.

Intracellular expression may be advantageous in some respects, *e.g.,* when using certain bacteria or fungi as host cells to produce large amounts of β-galactosidase for subsequent enrichment or purification. Extracellular secretion of β-galactosidase into the culture medium can also be used to make a cultured cell material comprising the isolated β-galactosidase.

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the β-galactosidase to a host cell organelle such as a peroxisome, or to a particular host cell compartment. Such a targeting sequence includes but is not limited to the sequence, SKL. For expression under the direction of control sequences, the nucleic acid sequence of the β-galactosidase is operably linked to the control sequences in proper manner with respect to expression.

The procedures used to ligate the DNA construct encoding a β-galactosidase, the promoter, terminator and other elements, respectively, and to insert them into suitable vectors containing the information necessary for replication, are well known to persons skilled in the art (see, *e.g.,* Sambrook et al., MOLECULAR CLONING: A LABORATORY MANUAL, 2nd ed., Cold Spring Harbor, 1989, and 3rd ed., 2001).

**Transformation and Culture of Host Cells**

An isolated cell, either comprising a DNA construct or an expression vector, is advantageously used as a host cell in the recombinant production of a β-galactosidase. The cell may be transformed with the DNA construct encoding the enzyme, conveniently by integrating the DNA construct (in one or more copies) in the host chromosome. This integration is generally considered to be an advantage, as the DNA sequence is more likely to be stably maintained in the cell. Integration of the DNA constructs into the host chromosome may be performed according to conventional methods, *e.g.,* by homologous or heterologous recombination. Alternatively, the cell may be transformed with an expression vector as described above in connection with the different types of host cells.

Examples of suitable bacterial host organisms are Gram positive bacterial species such as *Bacillaceae* including *Bacillus subtilis, Bacillus licheniformis, Bacillus lentus, Bacillus brevis, Geobacillus* (formerly *Bacillus*) *stearothermophilus, Bacillus alkalophilus, Bacillus amyloliquefaciens, Bacillus coagulans, Bacillus lautus, Bacillus megaterium,* and *Bacillus thuringiensis; Streptomyces* species such as *Streptomyces murinus;* lactic acid bacterial species including *Lactococcus* sp. such as *Lactococcus lactis; Lactobacillus* sp. including *Lactobacillus reuteri; Leuconostoc* sp.; *Pediococcus* sp.; and *Streptococcus* sp. Alternatively, strains of a Gram negative bacterial species belonging to *Enterobacteriaceae* including *E. coli,* or to *Pseudomonadaceae* can be selected as the host organism.

A suitable yeast host organism can be selected from the biotechnologically relevant yeasts species such as but not limited to yeast species such as *Pichia* sp., *Hansenula* sp., or *Kluyveromyces, Yarrowinia, Schizosaccharomyces* species or a species of *Saccharomyces,* including *Saccharomyces cerevisiae* or a species belonging to *Schizosaccharomyces* such as, for example, *S. pombe* species. A strain of the methylotrophic yeast species, *Pichia pastoris,* can be used as the host organism. Alternatively, the host organism can be a *Hansenula* species. Suitable host organisms among filamentous fungi include species of *Aspergillus, e.g., Aspergillus niger, Aspergillus oryzae, Aspergillus tubigensis, Aspergillus awamori,* or *Aspergillus nidulans.* Alternatively, strains of a *Fusarium* species, *e.g., Fusarium oxysporum* or of a *Rhizomucor* species such as *Rhizomucor miehei* can be used as the host organism. Other suitable strains include *Thermomyces* and *Mucor* species. In addition, *Trichoderma sp.* can be used as a host. A suitable procedure for transformation of *Aspergillus* host cells includes, for example, that described in EP 238023. A β-galactosidase expressed by a fungal host cell can be glycosylated, *i.e.,* will comprise a glycosyl moiety. The glycosylation pattern can be the same or different as present in the wild-type β-galactosidase. The type and/or degree of glycosylation may impart changes in enzymatic and/or biochemical properties.

It is advantageous to delete genes from expression hosts, where the gene deficiency can be cured by the transformed expression vector. Known methods may be used to obtain a fungal host cell having one or more inactivated genes. Gene inactivation may be accomplished by complete or partial deletion, by insertional inactivation or by any other means that renders a gene nonfunctional for its intended purpose, such that the gene is prevented from expression of a functional protein. A gene from a *Trichoderma* sp. or other filamentous fungal host that has been cloned can be deleted, for example, *cbh1, cbh2, egl1,* and *egl2* genes. Gene deletion may be accomplished by inserting a form of the desired gene to be inactivated into a plasmid by methods known in the art.

Introduction of a DNA construct or vector into a host cell includes techniques such as transformation; electroporation; nuclear microinjection; transduction; transfection, *e.g.*, lipofection mediated and DEAE-Dextrin mediated transfection; incubation with calcium phosphate DNA precipitate; high velocity bombardment with DNA-coated microprojectiles; and protoplast fusion. General transformation techniques are known in the art. *See, e.g.*, Sambrook *et al.* (2001), *supra.* The expression of heterologous protein in *Trichoderma* is described, for example, in U.S. Patent No. 6,022,725. Reference is also made to Cao et al. (2000) Science 9:991-1001 for transformation of *Aspergillus* strains. Genetically stable transformants can be constructed with vector systems whereby the nucleic acid encoding a β-galactosidase is stably integrated into a host cell chromosome. Transformants are then selected and purified by known techniques.

**Expression**

A method of producing a β-galactosidase may comprise cultivating a host cell as described above under conditions conducive to the production of the enzyme and recovering the enzyme from the cells and/or culture medium.

The medium used to cultivate the cells may be any conventional medium suitable for growing the host cell in question and obtaining expression of a β-galactosidase. Suitable media and media components are available from commercial suppliers or may be prepared according to published recipes (*e.g.*, as described in catalogues of the American Type Culture Collection).

An enzyme secreted from the host cells can be used in a whole broth preparation. In the present methods, the preparation of a spent whole fermentation broth of a recombinant microorganism can be achieved using any cultivation method known in the art resulting in the expression of a β-galactosidase. Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the β-galactosidase to be expressed or isolated. The term "spent whole fermentation broth" is defined herein as unfractionated contents of fermentation material that includes culture medium, extracellular proteins (*e.g.*, enzymes), and cellular biomass. It is understood that the term "spent whole fermentation broth" also encompasses cellular biomass that has been lysed or permeabilized using methods well known in the art.

An enzyme secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

The polynucleotide encoding a β-galactosidase in a vector can be operably linked to a control sequence that is capable of providing for the expression of the coding sequence by the host cell, *i.e.* the vector is an expression vector. The control sequences may be modified, for example by the addition of further transcriptional regulatory elements to make the level of transcription directed by the control sequences more responsive to transcriptional modulators. The control sequences may in particular comprise promoters.

Host cells may be cultured under suitable conditions that allow expression of a β-galactosidase. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or Sophorose. Polypeptides can also be produced recombinantly in an *in vitro* cell-free system, such as the TNT™ (Promega) rabbit reticulocyte system.

**Methods for Enriching and Purifying β-galactosidases**

Fermentation, separation, and concentration techniques are well known in the art and conventional methods can be used in order to prepare a β-galactosidase polypeptide-containing solution.

After fermentation, a fermentation broth is obtained, the microbial cells and various suspended solids, including residual raw fermentation materials, are removed by conventional separation techniques in order to obtain a β-galactosidase solution. Filtration, centrifugation, microfiltration, rotary vacuum drum filtration, ultrafiltration, centrifugation followed by ultra-filtration, extraction, or chromatography, or the like, are generally used.

It is desirable to concentrate a β-galactosidase polypeptide-containing solution in order to optimize recovery. Use of unconcentrated solutions requires increased incubation time in order to collect the enriched or purified enzyme precipitate.

The enzyme containing solution is concentrated using conventional concentration techniques until the desired enzyme level is obtained. Concentration of the enzyme containing solution may be achieved by any of the techniques discussed herein. Exemplary methods of enrichment and purification include but are not limited to rotary drum vacuum filtration and/or ultrafiltration.

The β-galactosidases in certain embodiments of the invention may be derived from a *Streptococcus* species, *Leuconostoc* species or *Lactobacillus* species, for example. Examples of *Streptococcus* species from which the β-galactosidase may be derived include *S. salivarius, S. sobrinus, S. dentirousetti, S. downei, S. mutans, S. oralis, S. gallolyticus* and *S. sanguinis.* Examples of Leuconostoc species from which the β-galactosidase may be derived include *L. mesenteroides, L. amelibiosum, L. argentinum, L. carnosum, L. citreum, L. cremoris, L. dextranicum* and *L. fructosum.* Examples of Lactobacillus species from which the β-galactosidase may be derived include *L. acidophilus, L. delbrueckii, L. helveticus, L. salivarius, L. casei, L. curvatus, L. plantarum, L. sakei, L. brevis, L. buchneri, L. fermentum* and *L. reuteri.*

## Pasteurization time and temperatures

Pasteurization of the milk-base at: ultra-high temperature (UHT) is normally carried out at 135-150°C for 2-15 sec, higher-heat/shorter time (HHST) normally carried out at 85-115°C for 0.5-9 sec and High Temperature/Short Time (HTST) pasteurization normally carried out at 70-85°C for 15-30sek. Pasteurization of Yogurt milk base is normally carried out at 85°C-96°C for 5-10 min.

## Preferred Embodiments of the Invention

A method is presented for determining carbohydrate content in a sample, the method having the steps of: obtaining FTIR (Fourier Transform Infrared Spectroscopy) spectrum data corresponding to the sample; providing at least a portion of the FTIR spectrum data as an input to a trained machine learning model; and processing the at least a portion of the FTIR spectrum data using the trained machine learning model to generate a carbohydrate content value providing a quantitative indication of a level of carbohydrate content in the sample.

Preferably, the carbohydrate is one or more of GOS, DP3+ GOS, glucose, galactose, and DP2. Preferably, DP2 is lactose. More preferably, the carbohydrate is DP3+ GOS.

Preferably, the sample is a milk-based substrate. Preferably, the portion of the FTIR spectrum data supplied as the input to the trained machine learning model is FTIR spectrum data within a limited spectral range. Preferably, the limited spectral range includes $1046cm^{-1}$, $1076cm^{-1}$, $1157\ cm^{-1}$ and $1250\ cm^{-1}$. More preferably, the limited spectral range is a wavenumber region for which a lower bound is between $900\ cm^{-1}$ and $1100\ cm^{-1}$ and an upper bound is between $1300\ cm^{-1}$ and $1500\ cm^{-1}$. Still more preferably, the limited spectral range is a wavenumber region for which a lower bound is between $1008\ cm^{-1}$ and $1068\ cm^{-1}$ and an upper bound is between $1414\ cm^{-1}$ and $1475\ cm^{-1}$. In the most preferred embodiments, the limited spectral range is in wavenumber region $1037:1450\ cm^{-1}$.

Preferably, the trained machine learning model is a supervised learning model trained using a training data set having, for each of a plurality of training samples, the FTIR spectrum data corresponding to the training sample and a measured indication of the level of carbohydrate content in the training sample.

Preferably, the trained machine learning model is a partial least squares regression (PLSR) model.

Preferably, the trained machine learning model is Neural Network regression model.

Preferably, the trained machine learning model comprises multiple-linear regression (MLR).

Preferably, the trained machine learning model is principle components regression (PCR).

Preferably, the trained machine learning model comprises classical least squares method CLS.

Preferably, the FTIR spectrum data is obtained from a server-based data store to which the FTIR spectrum data is uploaded by a client device.

Preferably, the processing of the at least a portion of the FTIR spectrum data using the trained machine learning model is performed at a server device using the FTIR spectrum data obtained from a client device.

Preferably, the carbohydrate content value is made accessible to the client device.

A method is presented for training a machine learning model to predict carbohydrate content in a milk-based substrate; the method having the steps of: obtaining a training data set having, for each of a plurality of training samples, FTIR (Fourier Transform Infrared Spectroscopy) spectrum data corresponding to the training sample and a measured indication of a level of carbohydrate content in the training sample; and performing supervised learning using the training data set, to determine trained model coefficients for the machine learning model.

Preferably, the carbohydrate is one or more of GOS, DP3+ GOS, glucose, galactose, and DP2. Preferably, the DP2 is lactose. Preferably, the carbohydrate is DP3+ GOS.

In another aspect of the invention, a computer program is presented which, when executed on a data processing apparatus, controls the data processing apparatus to perform the method as described above.

In another aspect of the present invention, a method for preparing a milk product containing carbohydrate is presented, having the steps of: treating a milk-based substrate with a trans-galactosylating enzyme; performing FTIR (Fourier Transform Infrared Spectroscopy) on a sample of the milk-based substrate to obtain FTIR spectrum data corresponding to the sample; obtaining, based on processing of at least a portion of the FTIR spectrum data using a trained machine learning model, a carbohydrate content value providing a quantitative indication of a level of carbohydrate content in the sample; and determining, based on the carbohydrate content value, when to inactivate the trans-galactosylating enzyme by pasteurization of the milk base.

Preferably, the carbohydrate is one or more of GOS, DP3+ GOS, glucose, galactose, and DP2. Preferably, the DP2 is lactose. Preferably, the carbohydrate is DP3+ GOS. 29.

Preferably, at least a portion of the FTIR spectrum data is uploaded by a client device to a server for server-based processing by the trained machine learning model to generate the carbohydrate content value, and the carbohydrate content value is obtained by the client device as a result of the server-based processing.

Preferably, the method for preparing a milk based carbohydrate has an accuracy better than 10%, expressed as Standard Error of Prediction at mean value (3.75 %), the concentration of GOS in a concentration range of 0-7.5 % in a milk base containing at least 0.1% fat, at least 0.5% dissolved lactose, and at least 1% protein.

Preferably, the method for preparing a milk based carbohydrate has a linearity (R2) of the PLS regression model above 0.9 (less preferred 0.85, 0.8, 0.75 and so forth) to validate the GOS content.

Preferably, the trans-galactosylating enzyme is derived from *Bifidobacterium bifidum.* More preferably, the transgalactosylating enzyme is a truncated β-galactosidase from *Bifidobacterium bifidum.* Yet more preferably, the truncated β-galactosidase from *Bifidobacterium bifidum* is truncated on the C-terminus. Still more preferably, the truncated β-galactosidase from *Bifidobacterium bifidum* is a polypeptide having at least 70% sequence identity to SEQ ID. NO: 1, SEQ ID NO:2, SEQ ID NO:3, SEQ ID NO:4 or SEQ ID NO:5 or to a transgalactosylase active fragment thereof.

Preferably, the polypeptide has at least 80% sequence identity to SEQ ID. NO:1, SEQ ID NO:2, SEQ ID NO:3, SEQ ID NO:4 or SEQ ID NO:5 or to a transgalactosylase active fragment thereof. More preferably, the polypeptide has at least 90% sequence identity to SEQ ID. NO:1, SEQ ID NO:2, SEQ ID NO:3, SEQ ID NO:4 or SEQ ID NO:5 or to a transgalactosylase active fragment thereof. Still more preferably, the polypeptide has at least 95% sequence identity to SEQ ID. NO:1, SEQ ID NO:2, SEQ ID NO:3, SEQ ID NO:4 or SEQ ID NO:5 or to a transgalactosylase active fragment thereof. In yet more preferred embodiments, the polypeptide has at least 99% sequence identity to SEQ ID. NO:1, SEQ ID NO:2, SEQ ID NO:3, SEQ ID NO:4 or SEQ ID NO:5 or to a transgalactosylase active fragment thereof. Still more preferably, the polypeptide is a sequence according to SEQ ID. NO:1, SEQ ID NO:2, SEQ ID NO:3, SEQ ID NO:4 or SEQ ID NO:5 or to a transgalactosylase active fragment thereof. In the most preferred embodiments, the polypeptide is a sequence according to SEQ ID NO: 1

In the method for preparing a milk product containing carbohydrate, preferably a PLS model with Linearity (R2) above 0.9 (less preferred 0.85, 0.8, 0.75 and so forth) is applied prior to pasteurization.

In the method for preparing a milk product containing carbohydrate, it is preferred that milk-based substrates having more than 20 % total solids are diluted in water before applying the infrared absorption measuring technique.

Preferably, the milk-based substrate has a lactose concentration of between 1-60% (w/w); or 2-50 % (w/w), or 3-40 % (w/w); or 4-30 % (w/w).

Preferably, the deactivation of the transgalactosylating enzyme is by heat treatment. More preferably, heat treatment is from about 70°C to 95°C and for between about 5 minutes to 30 minutes. Still more preferably, the heat treatment is at about 95°C for 5 to 30 minutes. In other preferred embodiments, the heat treatment is from about 135°C to about 150°C for about 2 seconds to about 15 seconds.

Preferably, the milk-based product having GOS fiber is yoghurt, ice cream, UHT milk, flavored milk product, concentrated/condensed milk product, milk-based powder, or cheese. Preferably, the GOS fiber in the milk-based product is stable having a variance of less than about 10% within 28 days. Preferably, the milk-based product having GOS fiber contains more than about 1.5 % (w/w) GOS fiber. More preferably, the milk-based product having GOS fiber contains more than about 3.2 % (w/w) GOS fiber. Still more preferably, the milk-based product having GOS fiber contains more than about 4 % (w/w) GOS fiber. In yet more preferred embodiments, the milk-based product having GOS fiber contains more than about 7 % (w/w) GOS fiber. Still more preferably, the milk-based product having GOS fiber contains more than about 14 % (w/w) GOS fiber. In yet more preferred embodiments, the milk-based product having GOS fiber contains more than about 30 % (w/w) GOS fiber.

Preferably the method is carried out by use of a full mid-infrared spectrum instrument arranged for recording a spectrum comprising the spectral range from about 400-6000 more specifically at least 900-1500 cm$^{-1}$.

Preferably, the Fourier Transform infrared (FTIR) Spectroscopy is carried out by use of a full mid-infrared spectrum instrument in order to obtain recorded spectral data comprising sufficient information. Preferably the recorded spectrum includes the spectral range from about 900 - 1500 cm$^{-1}$ or at least substantial wavebands thereof. Preferably, the full spectrum instrument includes data processing means for analyzing the spectral data. Alternatively, the spectral data might be transferred to remote data processing means arranged to perform a calculation of the GOS content from the spectrum. Among the advantages of the methods according to the present invention is that the methods may be carried out on a great number of instruments already located in laboratories all over the world. A further advantage is that a method according to the invention is more rapid than the known methods.

In other preferred embodiments of the present invention, the method further has the steps of dehydrating the low lactose milk-based product to provide a powder and dissolving the powder in water.

The present disclosure is described in further detail in the following examples, which are not in any way intended to limit the scope of the disclosure as claimed. The attached figures are meant to be considered as integral parts of the specification and description of the disclosure. The following examples are offered to illustrate, but not to limit the claimed disclosure.

The present invention is also described in the following aspects:

1. A method for determining carbohydrate content in a sample, the method comprising:

   obtaining FTIR (Fourier Transform Infrared Spectroscopy) spectrum data corresponding to the sample;
   providing at least a portion of the FTIR spectrum data as an input to a trained machine learning model; and
   processing at least a portion of the FTIR spectrum data using the trained machine learning model to generate a carbohydrate content value providing a quantitative indication of a level of carbohydrate content in the sample.

2. The method of aspect 1 wherein the carbohydrate is one or more of GOS, DP3+ GOS, glucose, galactose, and DP2.

3. The method of aspect 2 wherein the DP2 is lactose.

4. The method of aspect 2 wherein the carbohydrate is DP3+ GOS.

5. The method according to any of the previous aspects wherein the sample is a milk-based substrate.

6. The method according to any of the previous aspects, in which the portion of the FTIR spectrum data supplied as the input to the trained machine learning model comprises FTIR spectrum data within a limited spectral range.

7. The method of aspect 6, in which the limited spectral range includes 1046cm$^{-1}$, 1076cm$^{-1}$, 1157 cm$^{-1}$ and 1250 cm$^{-1}$.

8. The method aspect 6, in which the limited spectral range comprises a wavenumber region for which a lower bound is between 900 cm$^{-1}$ and 1100 cm$^{-1}$ and an upper bound is between 1300 cm$^{-1}$ and 1500 cm$^{-1}$.

9. The method of aspect 8, in which the limited spectral range comprises a wavenumber region for which a lower bound is between 1008 cm$^{-1}$ and 1068 cm$^{-1}$ and an upper bound is between 1414 cm$^{-1}$ and 1475 cm$^{-1}$.

10. The method of aspect 9, in which the limited spectral range comprises wavenumber region 1037:1450 cm$^{-1}$.

11. The method of any of the preceding aspects, in which the trained machine learning model comprises a supervised learning model trained using a training data set comprising, for each of a plurality of training samples, the FTIR spectrum data corresponding to the training sample and a measured indication of the level of carbohydrate content in the training sample.

12. The method of any of aspects 1 to 10, in which the trained machine learning model comprises a partial least squares regression (PLSR) model.

13. The method of any of aspects 1 to 10, in which the trained machine learning model comprises Neural Network regression model.

14. The method of any of aspects 1 to 10, in which the trained machine learning model comprises multiple-linear regression (MLR).

15. The method of any of aspects 1 to 10, in which the trained machine learning model comprises principle components regression (PCR).

16. The method of any of aspects 1 to 10, in which the trained machine learning model comprises classical least squares method CLS.

17. The method according to any of the previous aspects, in which the FTIR spectrum data is obtained from a server-based data store to which the FTIR spectrum data is uploaded by a client device.

18. The method according to any of the previous aspects, in which the processing of the at least a portion of the FTIR spectrum data using the trained machine learning model is performed at a server device using the FTIR spectrum data obtained from a client device.

19. The method of any of aspects 17 and 18, in which the carbohydrate content value is made accessible to the client device.

20. A method for training a machine learning model to predict carbohydrate content in a milk-based substrate; the method comprising:

obtaining a training data set comprising, for each of a plurality of training samples, FTIR (Fourier Transform Infrared Spectroscopy) spectrum data corresponding to the training sample and a measured indication of a level of carbohydrate content in the training sample; and
performing supervised learning using the training data set, to determine trained model coefficients for the machine learning model.

21. The method of aspect 20 wherein the carbohydrate is one or more of GOS, DP3+ GOS, glucose, galactose, and DP2.

22. The method of aspect 21 wherein the DP2 is lactose.

23. The method of aspect 21 wherein the carbohydrate is DP3+ GOS.

24. A computer program which, when executed on a data processing apparatus, controls the data processing apparatus to perform the method of any of the previous claims.

25. A method for preparing a milk product containing carbohydrate, comprising:

treating a milk-based substrate with a trans-galactosylating enzyme;
performing FTIR (Fourier Transform Infrared Spectroscopy) on a sample of the milk-based substrate to obtain FTIR spectrum data corresponding to the sample;
obtaining, based on processing of at least a portion of the FTIR spectrum data using a trained machine learning model, a carbohydrate content value providing a quantitative indication of a level of carbohydrate content in the sample; and
determining, based on the carbohydrate content value, when to inactivate the trans-galactosylating enzyme by pasteurization of the milk base.

26. The method of aspect 25 wherein the carbohydrate is one or more of GOS, DP3+ GOS, glucose, galactose, and DP2.

27. The method of aspect 26 wherein the DP2 is lactose.

28. The method of aspect 26 wherein the carbohydrate is DP3+ GOS.

29. The method of any of aspects 25 to 28, in which at least a portion of the FTIR spectrum data is uploaded by a client device to a server for server-based processing by the trained machine learning model to generate the carbohydrate content value, and the carbohydrate content value is obtained by the client device as a result of the server-based processing.

30. The method of any of aspects 25 to 29 having an accuracy better than 10%, expressed as Standard Error of Prediction at mean value (3.75 %), the concentration of GOS in a concentration range of 0-7.5 % in a milk base containing at least 0.1% fat, at least 0.5% dissolved lactose, and at least 1% protein.

31. The method of any of aspects 25 to 29 having a linearity ($R2$) of the PLS regression model above 0.9 (less preferred 0.85, 0.8, 0.75 and so forth) to validate the GOS content.

32. The method of any of aspects 25 to 31, in which the trans-galactosylating enzyme is derived from *Bifidobacterium bifidum.*

33. The method of aspect 32 wherein the transgalactosylating enzyme is a truncated β-galactosidase from *Bifidobacterium bifidum*.

34. The method of aspect 33 wherein the truncated β-galactosidase from *Bifidobacterium bifidum* is truncated on the C-terminus.

35. The method of aspect 34 wherein the truncated β-galactosidase from *Bifidobacterium bifidum* comprises a polypeptide having at least 70% sequence identity to SEQ ID. NO:1, SEQ ID NO:2, SEQ ID NO:3, SEQ ID NO:4 or SEQ ID NO:5 or to a transgalactosylase active fragment thereof.

36. The method of aspect 35 wherein the polypeptide has at least 80% sequence identity to SEQ ID. NO:1, SEQ ID NO:2, SEQ ID NO:3, SEQ ID NO:4 or SEQ ID NO:5 or to a transgalactosylase active fragment thereof.

37. The method of aspect 36 wherein the polypeptide has at least 90% sequence identity to SEQ ID. NO:1, SEQ ID NO:2, SEQ ID NO:3, SEQ ID NO:4 or SEQ ID NO:5 or to a transgalactosylase active fragment thereof.

38. The method of aspect 37 wherein the polypeptide has at least 95% sequence identity to SEQ ID. NO:1, SEQ ID NO:2, SEQ ID NO:3, SEQ ID NO:4 or SEQ ID NO:5 or to a transgalactosylase active fragment thereof.

39. The method of aspect 38 wherein the polypeptide has at least 99% sequence identity to SEQ ID. NO:1, SEQ ID NO:2, SEQ ID NO:3, SEQ ID NO:4 or SEQ ID NO:5 or to a transgalactosylase active fragment thereof.

40. The method of aspect 39 wherein the polypeptide comprises a sequence according to SEQ ID. NO:1, SEQ ID NO:2, SEQ ID NO:3, SEQ ID NO:4 or SEQ ID NO:5 or to a transgalactosylase active fragment thereof.

41. The method of aspect 40 wherein the polypeptide comprises a sequence according to SEQ ID. NO: 1.

## EXAMPLES

### Example 1: HPLC lactose determination

The following method describes the procedure for lactose quantification in milk samples from example 4-8. The samples were derivatized and analyzed by HPLC with UV and FLD detection.

Chemicals: Dimethyl sulfoxide, DMSO (CAS: 67-68-5 Sigma A8418); Phosphoric acid, H3PO4, (CAS: 7664-38-2, Sigma P5811); Acetic acid, ≥99 % (CAS: 64-19-7, Sigma A6283); Sodium phosphate, $NaH_2PO_4$, ≥99.0 % (CAS: 7558-79-4, Sigma S7907); 4-Aminobenzoic acid, ≥99 % (CAS: 150-13-0, Sigma A9878); 2-Methylpyridine borane complex solution, 95 % (CAS: 3999-38-0, Sigma 654213); Tetrabutylammonium bisulfate, ≥99.0 %, (CAS: 32503-27-8, Sigma 86868); Lactose (CAS: 10039-26-6, Sigma)

The derivatization reagent was obtained by mixing 12.3 % w/v 4-aminobenzoic acid and 10 % w/v 2-methylpyridine borane in DMSO/acetic acid (70/30 % v/v). The sample solvent was 10 mM $Na_2HPO_4$ (adjusted to pH 2.5 with $H_3PO_4$ (85 %)).

A ThermoScientific UltiMate 3000 HPLC system equipped with online vacuum degasser, pump, autosampler, column temperature control and UV/VIS-detector was utilized. ThermoScientific Chomeleon 7.2.9 software was used for quantification. Column: Knauer Prontosil RP-C18 SH (150x4.6 mm, 3 μm) from Mikrolab (KN 15EF180PSG /15VH185PSJ). Tetrabutylammonium hydrogen sulphate was used as the ion-pair reagent in the eluent system. Injection volume was 20 μL, column temperature 20 °C, Isocratic: MP A Flow: 0.8 mL/min, A: 10 mM sodium phosphate buffer containing 20 mM tetrabutylammonium bisulfate (pH 2.0). In between each injection column was washed with 50/50 % v/v acetonitrile/water, Pressure: 250 bar, Runtime: 100 min, Detector absorbance measurement at 303 nm. Lactose used for calibration standard was made ranging from 5-500 mg/L in $ddH_2O$.

Sample preparation: L-Arabinose (75 mg/L) prepared in 10 mM $NaH_2PO_4$ at pH 2.5 was utilized as internal standard for all samples. For the milk and standards: 200 μL sample/standard was transferred to a 1.5 mL Eppendorf tube, added 400 μL sample solvent and mixed. This mixture was centrifuged for 10 min. at max speed (Labnet centrifuge) and 200 μL of the supernatant was transferred to a 2 mL Eppendorf tube. The samples were derivatized as follows: To the 2 mL Eppendorf tube containing 200 μL sample, 200 μL derivatization reagent was added. The tube was placed in a Thermomixer at 60 °C to react for 30 min. shaking at 750 rpm. Afterwards, 25 μL of the reaction mixture was mixed with 225 μL mL mobile phase in a microtiter plate. This solution was filtered through a microtiter filter-plate (Corning filter plate, PVDF hydrophile membrane, NY USA, 0.2 μm) by centrifugation (10 000 rpm; 10 min). The plate was sealed before the final HPLC analysis. The lactose concentration in the dairy samples (including milk) was calculated according to the calibration standards and internal control.

**Example 2: HPLC method for quantification of GOS fiber**

The standard lactose (HPLC analytical grade, Sigma Aldrich) was prepared in double distilled water (ddH$_2$O). A dilution series ranging from 500 to 10000 ppm of the lactose standard was prepared. The milk samples from example 4-8 were weighed and diluted using ddH$_2$O to approximately 5 % milk carbohydrate. A very limited number of samples were pipetted assuming a sample density of 1.0 g/ml. Afterwards, 200 mg sample in 800 μL H$_2$O was mixed thoroughly and 50 μL Carrez reagent A (Carrez Clarification Kit, 1.10537.001, Merck) and 50 μL Carrez B were added with mixing after each addition to induce protein and lipid precipitation. The mixture was incubated 15 minutes at room temperature. The sample was centrifuged at 10.000 rpm for 4 min and 300 μL clarified supernatant was filtered through an MTP 96 well-filter plate (Corning filter plate, PVDF hydrophile membrane, NY USA) with a pore size of 0.20 μm (centrifuged 3000 rpm in 15 minutes) before analysis. All samples were analyzed in duplicate and in 96 well MTP plates sealed with tape.

Instrumentation:

Quantification of higher galacto-oligosaccharides (GOS, ie. DP3 and above), DP2, glucose and galactose were performed by HPLC. Analysis of samples was carried out on a Dionex Ultimate 3000 HPLC system (Thermo Fisher Scientific) equipped with a DGP-3600SD Dual-Gradient analytical pump, WPS-3000TSL thermostated autosampler, TCC-3000SD thermostated column oven, and a RI-101 refractive index detector (Shodex, JM Science). Chromeleon datasystem software (Version 6.80, DU10A Build 2826, 171948) was used for data acquisition and analysis.

Chromatographic conditions:

The samples were analyzed by HPLC using an RSO oligosaccharide column, Ag$^+$ 4 % crosslinked (Phenomenex, The Netherlands) equipped with an analytical guard column (Carbo-Ag$^+$ neutral, AJ0-4491, Phenomenex, The Netherlands) operated at 70°C. The column was eluted with double distilled water at a flow rate of 0.3 ml/min.
Isocratic flow of 0.3 ml/min was maintained throughout analysis with a total run time of 45 minutes. The injection volume was set to 10 μL. Samples were held at 30°C in the thermostated autosampler compartment to ensure solubilization of all components. The eluent was monitored by means of a refractive index detector (RI-101, Shodex, JM Science) and quantification was made by the peak area relative to the peak area of lactose standards as described above. Peaks of DP 3 and higher (DP3+) were quantified as galacto-oligosaccharides (DP3, DP4, DP5 and so forth). The assumption that all DP3+ galacto-oligosaccharides components provide same response was confirmed with mass balances. DP2 species including lactose, glucose and galactose were all quantified respectively against the lactose standards as described above.

**Example 3: Total GOS**

Total GOS (TGOS) is defined as the total sum of transgalactosylated molecules. The value is calculated based of the amount of galactose bound in TGOS molecules multiplied with a factor of 1.4 in which the factor of 1.4 represents the galactose to glucose ratio. This will include a larger fraction of trans-galactosylated disaccharides such as allolactose (β-D-Galactopyranosyl (1→6)-D-glucose), (β-D-Galactopyranosyl (1→3)-D-glucose) or (β-D-Galactopyranosyl (1→4)-D-glacatose). The galactose bound in TGOS molecules is calculated by subtracting free galactose and galactose bound in lactose from the total galactose (which is the sum of free galactose, galactose bound in TGOS and galactose bound in lactose). This calculation approach is in line with AOAC 2001.02 method.
Based on values obtained from example 1 and output values from the method of example 2 we calculate the TGOS as follows:

$$\text{TGOS} = (\text{Total carb. (\% w/w)} / 1.9 - \text{galactose (\% w/w)} - \text{Lactose (\% w/w)} / 1.9) \times 1.4$$

Total carb. (% w/w) is the sum of DP3+ (% w/w), DP2 (% w/w), glucose (% w/w) and galactose (% w/w).
Total carb. (% w/w) / 1.9 represents total galactose in the sample
Glucose (% w/w) is the free glucose in the sample
Galactose (% w/w) is the free galactose in the sample.
Lactose (% w/w) / 1.9 is the galactose bound in the lactose molecule

**Example 4: In situ production of GOS in low fat milk base of 3% protein**

Milk bases were prepared for enzyme reaction with the GOS producing *Bifidobacterium bifidum* β-galactosidase, SEQ ID No. 1, for in situ GOS generation. Skimmed milk (Arla Foods, Denmark, 0.1 % fat, 4.7 % lactose, 3.66 % protein) was standardized to 3 % protein with tap water and used as is (resulting in 3.8% lactose) or adjusted to either 4.7 %, 6 % or 8 % lactose with Variolac® 992 BG100 (Arla Foods, Denmark). The enzyme Zymstar GOS (Material A15017, batch 4863445828) was dosed according to table 1 and each sample was placed at 5 °C for 18 hours. After 18 hours, 250 ml sample of each was heat treated in a water bath for 15 minutes at 95 °C. The resulting samples were subjected to Fourier Transform infrared (FTIR) Spectroscopy and the individual carbohydrates in the samples were quantified according to example 1 and 2.

Table 1

| Sample ID | lactose (% w/v) | Protein (% w/v) | fat (% w/v) | Enzyme (g/L) |
|---|---|---|---|---|
| 1 | 3.86 | 3 | 0.08 | 0 |
| 2 | 3.86 | 3 | 0.08 | 0.8 |
| 3 | 3.86 | 3 | 0.08 | 1.9 |
| 4 | 3.86 | 3 | 0.08 | 2.4 |
| 5 | 3.86 | 3 | 0.08 | 3.4 |
| 6 | 3.86 | 3 | 0.08 | 4.8 |
| 7 | 4.7 | 3 | 0.08 | 0 |
| 8 | 4.7 | 3 | 0.08 | 1 |
| 9 | 4.7 | 3 | 0.08 | 2.5 |
| 10 | 4.7 | 3 | 0.08 | 3.2 |
| 11 | 4.7 | 3 | 0.08 | 4.5 |
| 12 | 4.7 | 3 | 0.08 | 6.4 |
| 13 | 6 | 3 | 0.08 | 0 |
| 14 | 6 | 3 | 0.08 | 1.2 |
| 15 | 6 | 3 | 0.08 | 3 |
| 16 | 6 | 3 | 0.08 | 3.9 |
| 17 | 6 | 3 | 0.08 | 5.5 |
| 18 | 6 | 3 | 0.08 | 7.8 |
| 19 | 8 | 3 | 0.08 | 0 |
| 20 | 8 | 3 | 0.08 | 1.6 |
| 21 | 8 | 3 | 0.08 | 4 |
| 22 | 8 | 3 | 0.08 | 5.1 |
| 23 | 8 | 3 | 0.08 | 7.2 |
| 24 | 8 | 3 | 0.08 | 10.2 |

**Example 5: In situ production of GOS in milk base of 6% protein**

Milk bases were prepared for enzyme reaction with the GOS producing *Bifidobacterium bifidum* β-galactosidase, SEQ ID No. 1, for in situ GOS generation. A skimmed milk UF concentrate (10.07 % w/v protein and 0.09 % w/v fat) was standardized to 5.7 % w/v protein with tap water and added either 3 %, 4.7 %, 6 % or 8 % lactose with Variolac® 992 BG100 (Arla Foods). The enzyme Zymstar GOS (Material A15017, batch 4863445828) was dosed according to table 2 and each sample was placed at 5 °C for 18 hours. After 18 hours, 250 ml sample of each was heat treated in a water bath for 15 minutes at 95 °C. The resulting samples were subjected to Fourier Transform infrared (FTIR) Spectroscopy and the individual carbohydrates in the samples were quantified according to example 1 and 2.

Table 2

| Sample ID | lactose (%) | Protein (%) | fat (%) | Enzyme (g/L) |
|---|---|---|---|---|
| 25 | 5 | 5.7 | 0.05 | 0 |
| 26 | 5 | 5.7 | 0.05 | 0.8 |
| 27 | 5 | 5.7 | 0.05 | 1.9 |
| 28 | 5 | 5.7 | 0.05 | 2.4 |
| 29 | 5 | 5.7 | 0.05 | 3.4 |
| 30 | 5 | 5.7 | 0.05 | 4.8 |
| 31 | 6.7 | 5.7 | 0.05 | 0 |
| 32 | 6.7 | 5.7 | 0.05 | 1 |
| 33 | 6.7 | 5.7 | 0.05 | 2.5 |
| 34 | 6.7 | 5.7 | 0.05 | 3.2 |
| 35 | 6.7 | 5.7 | 0.05 | 4.5 |
| 36 | 6.7 | 5.7 | 0.05 | 6.4 |
| 37 | 8 | 5.7 | 0.05 | 0 |
| 38 | 8 | 5.7 | 0.05 | 1.2 |
| 39 | 8 | 5.7 | 0.05 | 3 |
| 40 | 8 | 5.7 | 0.05 | 3.9 |
| 41 | 8 | 5.7 | 0.05 | 5.5 |
| 42 | 8 | 5.7 | 0.05 | 7.8 |
| 43 | 10 | 5.7 | 0.05 | 0 |
| 44 | 10 | 5.7 | 0.05 | 1.6 |
| 45 | 10 | 5.7 | 0.05 | 4 |
| 46 | 10 | 5.7 | 0.05 | 5.1 |
| 47 | 10 | 5.7 | 0.05 | 7.2 |
| 48 | 10 | 5.7 | 0.05 | 10.2 |

Example 6: **In** situ production of GOS in skimmed milk with various lactose levels

Milk bases were prepared for enzyme reaction with the GOS producing *Bifidobacterium bifidum* β-galactosidase, SEQ ID No. 1, for in situ GOS generation. Arla skimmed milk (4.7 % lactose, 3.66 % protein and 0.09 % fat) was standardized to 3% lactose by dilution with tap water or standardized to 4.7 %, 6 % or 8 % lactose with Variolac® 992 BG100 (Arla Foods). The enzyme Zymstar GOS (Material A15017, batch 4863445828) was dosed according to table 3 and each sample was placed at 5 °C for 18 hours. After 18 hours, 250 ml sample of each was heat treated in a water bath for 15 minutes at 95 °C. The resulting samples were subjected to Fourier Transform infrared (FTIR) Spectroscopy and the individual carbohydrates in the samples were quantified according to example 1 and 2.

Table 3

| Sample ID | lactose (%) | Protein (%) | fat (%) | Enzyme (g/L) |
|---|---|---|---|---|
| 49 | 3 | 2.3 | 0.05 | 0 |
| 50 | 3 | 2.3 | 0.05 | 0.8 |
| 51 | 3 | 2.3 | 0.05 | 1.9 |

(continued)

| Sample ID | lactose (%) | Protein (%) | fat (%) | Enzyme (g/L) |
|---|---|---|---|---|
| 52 | 3 | 2.3 | 0.05 | 2.4 |
| 53 | 3 | 2.3 | 0.05 | 3.4 |
| 54 | 3 | 2.3 | 0.05 | 4.8 |
| 55 | 4.7 | 3.6 | 0.1 | 0 |
| 56 | 4.7 | 3.6 | 0.1 | 1 |
| 57 | 4.7 | 3.6 | 0.1 | 2.5 |
| 58 | 4.7 | 3.6 | 0.1 | 3.2 |
| 59 | 4.7 | 3.6 | 0.1 | 4.5 |
| 60 | 4.7 | 3.6 | 0.1 | 6.4 |
| 61 | 6 | 3.6 | 0.1 | 0 |
| 62 | 6 | 3.6 | 0.1 | 1.2 |
| 63 | 6 | 3.6 | 0.1 | 3 |
| 64 | 6 | 3.6 | 0.1 | 3.9 |
| 65 | 6 | 3.6 | 0.1 | 5.5 |
| 66 | 6 | 3.6 | 0.1 | 7.8 |
| 67 | 8 | 3.6 | 0.1 | 0 |
| 68 | 8 | 3.6 | 0.1 | 1.6 |
| 69 | 8 | 3.6 | 0.1 | 4 |
| 70 | 8 | 3.6 | 0.1 | 5.1 |
| 71 | 8 | 3.6 | 0.1 | 7.2 |
| 72 | 8 | 3.6 | 0.1 | 10.2 |

**Example 7: In situ production of GOS in full fat milk base of 3% protein**

Milk bases were prepared for enzyme reaction with the GOS producing *Bifidobacterium bifidum* β-galactosidase, SEQ ID No. 1, for in situ GOS generation. Arla skimmed milk (4.7 % lactose, 3.66 % protein and 0.09 % fat) was standardized to 3 % lactose and 3.5 % fat by addition of tap water and addition of Cream (Arla Foods, Denmark, 38 % fat). The Milk base was used as is or adjusted to 4.7 %, 6 % or 8 % lactose with Variolac® 992 BG100 (Arla Foods). The enzyme Zymstar GOS (Material A15017, batch 4863445828) was dosed according to table 4 and each sample was placed at 5 °C for 18 hours. After 18 hours, 250 ml sample of each was heat treated in a water bath for 15 minutes at 95 °C. The resulting samples were subjected to Fourier Transform infrared (FTIR) Spectroscopy and the individual carbohydrates in the samples were quantified according to example 1 and 2.

Table 4

| Sample ID | lactose (%) | Protein (%) | fat (%) | Enzyme (g/L) |
|---|---|---|---|---|
| 73 | 3 | 2.3 | 3.5 | 0 |
| 74 | 3 | 2.3 | 3.5 | 0.8 |
| 75 | 3 | 2.3 | 3.5 | 1.9 |
| 76 | 3 | 2.3 | 3.5 | 2.4 |
| 77 | 3 | 2.3 | 3.5 | 3.4 |
| 78 | 3 | 2.3 | 3.5 | 4.8 |

(continued)

| Sample ID | lactose (%) | Protein (%) | fat (%) | Enzyme (g/L) |
|---|---|---|---|---|
| 79 | 4.7 | 3.4 | 3.5 | 0 |
| 80 | 4.7 | 3.4 | 3.5 | 1 |
| 81 | 4.7 | 3.4 | 3.5 | 2.5 |
| 82 | 4.7 | 3.4 | 3.5 | 3.2 |
| 83 | 4.7 | 3.4 | 3.5 | 4.5 |
| 84 | 4.7 | 3.4 | 3.5 | 6.4 |
| 85 | 6 | 3.4 | 3.5 | 0 |
| 86 | 6 | 3.4 | 3.5 | 1.2 |
| 87 | 6 | 3.4 | 3.5 | 3 |
| 88 | 6 | 3.4 | 3.5 | 3.9 |
| 89 | 6 | 3.4 | 3.5 | 5.5 |
| 90 | 6 | 3.4 | 3.5 | 7.8 |
| 91 | 8 | 3.4 | 3.5 | 0 |
| 92 | 8 | 3.4 | 3.5 | 1.6 |
| 93 | 8 | 3.4 | 3.5 | 4 |
| 94 | 8 | 3.4 | 3.5 | 5.1 |
| 95 | 8 | 3.4 | 3.5 | 7.2 |
| 96 | 8 | 3.4 | 3.5 | 10.2 |

### Example 8: In situ production of GOS in high fat milk base of 3% protein

Milk bases were prepared for enzyme reaction with the GOS producing *Bifidobacterium bifidum* β-galactosidase, SEQ ID No. 1, for in situ GOS generation. A skimmed milk UF concentrate (10.07 % protein and 0.09 % fat) was standardized to 6 % protein and 3.5 % fat by dilution with tap water and addition of Cream (Arla Foods, Denmark, 38 % fat). The Milk base was used as is or adjusted to 4.7 %, 6 % or 8 % lactose with Variolac® 992 BG100 (Arla Foods). The enzyme Zymstar GOS (Material A15017, batch 4863445828) was dosed according to table 5 and each sample was placed at 5 °C for 18 hours. After 18 hours, 250 ml sample of each was heat treated in a water bath for 15 minutes at 95 °C. The resulting samples were subjected to Fourier Transform infrared (FTIR) Spectroscopy and the individual carbohydrates in the samples were quantified according to example 1 and 2.

Tabel 5

| Sample ID | lactose (%) | Protein (%) | fat (%) | Enzyme (g/L) |
|---|---|---|---|---|
| 97 | 5.86 | 5.7 | 3.5 | 0 |
| 98 | 5.86 | 5.7 | 3.5 | 0.8 |
| 99 | 5.86 | 5.7 | 3.5 | 1.9 |
| 100 | 5.86 | 5.7 | 3.5 | 2.4 |
| 101 | 5.86 | 5.7 | 3.5 | 3.4 |
| 102 | 5.86 | 5.7 | 3.5 | 4.8 |
| 103 | 6.7 | 5.7 | 3.5 | 0 |
| 104 | 6.7 | 5.7 | 3.5 | 1 |
| 105 | 6.7 | 5.7 | 3.5 | 2.5 |

(continued)

| Sample ID | lactose (%) | Protein (%) | fat (%) | Enzyme (g/L) |
|-----------|-------------|-------------|---------|--------------|
| 106 | 6.7 | 5.7 | 3.5 | 3.2 |
| 107 | 6.7 | 5.7 | 3.5 | 4.5 |
| 108 | 6.7 | 5.7 | 3.5 | 6.4 |
| 109 | 8 | 5.7 | 3.5 | 0 |
| 110 | 8 | 5.7 | 3.5 | 1.2 |
| 111 | 8 | 5.7 | 3.5 | 3 |
| 112 | 8 | 5.7 | 3.5 | 3.9 |
| 113 | 8 | 5.7 | 3.5 | 5.5 |
| 114 | 8 | 5.7 | 3.5 | 7.8 |
| 115 | 10 | 5.7 | 3.5 | 0 |
| 116 | 10 | 5.7 | 3.5 | 1.6 |
| 117 | 10 | 5.7 | 3.5 | 4 |
| 118 | 10 | 5.7 | 3.5 | 5.1 |
| 119 | 10 | 5.7 | 3.5 | 7.2 |
| 120 | 10 | 5.7 | 3.5 | 10.2 |

**Example 9:**

**Fourier Transform infrared (FTIR) Spectroscopy- Perkin Elmer Spectrum One instrument:**

The infrared spectroscopy method is based on instruments recording the mid-infrared (MIR) region of the electromagnetic spectrum. Typically, the MIR infrared absorbance is measured in the wavenumber range of 650-4400 $cm^{-1}$.

A sample set of 10 GOS containing milk samples (selected from examples 4-8) was freeze-dried in-vacuo overnight and the powder was analyzed in triplicate using a Spectrum One FTIR Instrument (Perkin Elmer, Waltham MA USA) equipped with a universal attenuated total reflectance (UATR) unit using a spectral resolution of 4 $cm^{-1}$ and 64 scans in the spectral range 650-4400 $cm^{-1}$. The results are shown in Figure 1. The spectra were further treated and evaluated as described in example 10 for GOS concentration determination.

FIG. 1 shows the absorbance mid-infrared spectra from 10 multiple scatter corrected spectra of dried milk samples containing GOS analyzed by Perkin Elmer Spectrum One FTIR instrument in the wavenumber-range 650-4400 $cm^{-1}$ using a spectral resolution of 4 $cm^{-1}$. The black/white bar is shaded according to the GOS (DP3+) level as measured by HPLC (example 2) as shown on second axis in w/w %. Wavenumber regions: #1 (1037 -1445 $cm^{-1}$), #2 (650-4400 $cm^{-1}$) and #3 (650-1036 + 1449-4400 $cm^{-1}$) are marked in spectrum with lines (used for modelling in example 10). The optimal model (Model #1) is marked with two dotted lines.

**Example 10**

A Partial Least Squares Regression (PLSR)-model algorithm for prediction of the concentration of GOS in milk samples was derived. The principle of the algorithm is to use selected regions of the FTIR spectra to predict the GOS concentrations as measured by HPLC (described in example 2). The algorithm is based on multivariate mathematical model called Partial Least Squares (PLS) regression model, where many wavenumbers in the FTIR spectrum are used to create an optimal prediction of the HPLC result. Similar methodology was used to predict the glucose, galactose, DP2 and lactose as measured by example 1 and 2, however only the DP3+ GOS prediction is described in detail below. With the prediction of all the carbohydrates as measured in example 1 and 2 one would be able to apply example 3 to predict the total GOS concentration.

This type of model is needed as no single/nor a few wavenumbers in the FTIR spectrum can be used to quantify the GOS content. The FTIR spectrum reports on all milk constituents (Milk, fat and sugars) but by use of PLS Regression an optimal region for DP3+ GOS prediction was discovered.

The mathematical model PLS Regression is described elsewhere (Martens, Izquierdo, Thomassen, & Martens, 1986).

The PLSR-model was built and tested using the PLS-Toolbox 8.7 in Matlab 2019a. This implementation uses the NIPALS algorithm to estimate the weights, i.e. the PLS loadings.

A sample-set of 30 FTIR spectra (recorded as described in example 9) containing spectral information of GOS containing milk samples were collected and tabulated with the coherent HPLC DP3+ GOS results (as described in example 2).

Five wavenumber regions were used for modelling, resulting in Model #1 to #5 (Table 6). An optimal model (Model #1) was found using data-points ranging (region #1) from 1037-1445 cm$^{-1}$ (see Figure 1). The optimal number of PLS components/Latent Variables found was 4 for this small data-set as this number resulted in a low prediction error - as illustrated in Figure 2. A higher number of PLS components would also result in a low prediction error but choosing a higher number will result in overfitting a model based on 30 samples.

The model was validated using cross validation (Venetian blinds, 10 splits) and the reported model performances in Table 5 are the cross validated results. The Model #1 performance for the DP3+ GOS-prediction showed an R-squared $R^2 = 0.924$ and a root-mean-squared error of prediction RMSECV = 0.153.

Table 6:

| PLS Regression model comparison | Spectral range in wavenumbers cm$^{-1}$ | Linearity (R$^2$) of PLS regression model – should be close to 1.0 | Error (prediction) of PLS-regression model (RMSECV) – should be as low as possible | Comments | Latent variables |
|---|---|---|---|---|---|
| **Model #1** | 1037:1445 | 0.924 | 0.153 | Range used for the DP3+ model | 4 |
| **Model #2** | 650:4400 | 0.796 | 0.252 | Complete spectrum | 4 |
| **Model #3** | 650:1036 + 1449:4400 | 0.759 | 0.275 | Complete spectrum minus model range | 4 |
| **Model #4** | 1008:1475 | 0.861 | 0.207 | More broad spectral range +30cm-1 at each border | 4 |
| **Model #5** | 1068:1415 | 0.911 | 0.165 | More narrow spectral range minus 30cm-1 at each border | 4 |

The discovered model with the optimal spectral range (Model #1) has superior model performance compared to using the complete spectrum (Model #2). The performance is measured by the model R-squared, which should be as close as possible to 1.00 and the error (RMECV) which should be a low as possible. The spectral range in Model #1 is covering among others infrared absorbances from C-O-C (1157 and 1250cm$^{-1}$) present in oligosaccharides like GOS (Grelet, Fernández Pierna, Dardenne, Baeten, & Dehareng, 2015). To test if the discovered Model #1 was the optimum, two models were tested with ranges of 30 wavenumbers (cm-1) higher or lower. Model #4 and #5 were inferior in model performance compared to Model #1 - see Table 6.

Finally, another model (Model #3) with spectral ranges below and beyond Model #1 range have a much lower performance - illustrating that the discovered Model #1 covers the optimal spectral range for GOS enzyme activity prediction. All models reported in table 6 have 4 PLS components.

PLS model coefficients of model Model #1 are visualized in Figure 3. The regression Vector of Y1 (=DP3+) is depicted and model coefficients as a function of wavenumbers. The importance of each wavenumber to the prediction of DP3+ GOS is proportional to the model coefficients. The absolute value of the model coefficients indicates the relative importance of each wavenumber, while the sign of the coefficients indicates the sign of the contribution to the DP3+ GOS values.

**Example 11: Fourier Transform infrared (FTIR) Spectroscopy based on Foss MilkoScan FT2 instrument:**

To test the general implementation, another FTIR spectroscopy instrument was used. An instrument Foss MilkoScan FT2 controlled by the Foss Intergrator 2 software was used for the analysis. The apparatus was cleaned via the embedded cleaning program before every run. The zero-setting option of the Foss Integrator 2 program was used to control the success of each cleaning step. For the measurement, 100 mL sample were placed below the nozzle of the instrument. Each sample was analyzed 4 times. Placing a milk sample at the instrument the flow-tube inlet system samples two times (11 mL each time) and each sample is measured in duplicate.

The data were collected in two ways. First, as a summarized out-put of pre-evaluated standard data provided by the instrument was extracted. This out-put was used as a control for the fat, protein and lactose content.

Secondly, the unprocessed raw-data (FTIR spectra) of each run of the instrument were extracted from the Intergator2 software. Examples of spectral data from MilkoScan FT2 analyzing milk samples containing GOS (generated as described in example 4-8) are given in Figure 4. The FTIR spectra from the instrument software are given in data-points, not in wavenumbers. The FOSS MilkoScan spectrum runs over the interval 926:5010 cm$^{-1}$ (Grelet, et al., 2015), with data-points running over the interval 240:1299. Hence the conversion factor was calculated to be (5020-926)/(1299-240) = 3.856 cm$^{-1}$/data-point.

**Example 12: PLS Regression-model for prediction of DP3+ GOS in milk samples using MilkoScan FTIR Spectra.**

A Partial Least Squares Regression (PLSR)-model algorithm for prediction of the concentration of DP3+ GOS in milk samples was derived from Milkoscan data-files. The principle of the algorithm is to use selected regions of the FTIR spectra to predict the DP3+ GOS concentrations as measured by HPLC (described in example 2). The algorithm is based on multivariate mathematical model called Partial Least Squares (PLS) regression model, where many wavenumbers/data-points in the FTIR spectrum are used to create an optimal prediction of the HPLC result. Similar methodology was used to predict the glucose, galactose, DP2 and lactose as measured by example 1 and 2, however only the DP3+ GOS prediction is described in detail below. With the prediction of all the carbohydrates as measured in example 1 and 2 one would be able to apply example 3 to predict the total GOS concentration.

This type of model is needed as no single/nor a few wavenumbers in the FTIR spectrum can be used to quantify the DP3+ GOS content. The FTIR spectrum reports on all milk constituents (Milk, fat and sugars) but by use of PLS Regression an optimal region for DP3+ GOS prediction was discovered.

The mathematical model PLS Regression is described elsewhere (Martens, et al., 1986).

The PLSR-model was built and tested using the python function sklearn.pls.PLSRegression from the scikit-learn package v. 0.22.1. This implementation uses the NIPALS algorithm to estimate the weights, i.e. the PLS loadings.

A sample-set of 119 (produced as in examples 4-8) data-files containing spectral information of GOS containing milk samples were collected and tabulated with the coherent HPLC GOS (DP3+) results (as described in example 2). The 119 GOS containing milk samples were randomly divided into a test and a training set. The training set was used to build the model and the test set to validate the model. The training set contained 95 GOS containing milk samples, while 24 GOS containing milk samples were used to validate the PLSR model.

An optimal model (Model #6) was found using data-points ranging from 270-376. By conversion this data-point range correspond to wavenumbers 1041 cm$^{-1}$ -1450 cm$^{-1}$, see figure 5.

In Table 7, three different PLS Regression models are compared. The discovered model with the optimal spectral range (Model #6) has superior model performance compared to using the complete spectrum (Model #7).

Testing another model (Model #8) with ranges below and beyond Model #6 range have a much lower performance - illustrating that the discovered Model #6 covers the optimal spectral range. All models reported in Table 7 were allowed a maximum of 8 PLS components, the optimal number of components, within this max, were found by cross validation and is reported in the table.

The optimal number of PLS components in Model #6 found by cross validation was 13. However, 13 PLS components produces a slightly over-fitted model, sensitive to very small changes in the raw FTIR spectra. Therefore, a suboptimal model with 8 latent variables was chosen, as this was assessed to give the best tradeoff between model accuracy and model robustness.

The model was validated using the test-set of 24 GOS containing milk samples. The Model #6 performance for the DP3+ GOS-prediction showed an R-squared $R^2$ = 0.964 and a root-mean-squared error of prediction RMSEP = 0.19. The model accuracy is illustrated in Figure 6, which shows the predicted vs. the measured DP3+ GOS content in w/w %.

Table 7.

| PLS Regression model comparison | Spectral range in wavenumbers $cm^{-1}$ | Linearity ($R^2$) of PLS regression model – should be close to 1.0 | Error (prediction) of PLS-regression model (RMSEP) – should be as low as possible | Comments | Latent variables |
|---|---|---|---|---|---|
| Model #6 | 1041:1450 | 0.964 | 0.194 | Range used for the DP3+ model | 8 |
| Model #7 | 926:5010 | 0.841 | 0.407 | Complete spectrum | 8 |
| Model #8 | 926:1037 +1454:5010 | 0.799 | 0.459 | Complete spectrum minus model range | 8 |

PLS model coefficients of model Model #6 are visualized in Figure 7. The importance of each data-point to the prediction of DP3+ GOS is proportional to the model coefficients. The absolute value of the model coefficients indicates the relative importance of each data-point, while the sign of the coefficients indicates the sign of the contribution to the DP3+ GOS values. Figure 7 shows that most model coefficients are of same order of magnitude, but with a regional change in the sign. I.e. most data-points are of similar importance to the DP3+ GOS prediction but linked positively or negatively to DP3+ GOS depending on region. As the absorbance at subsequent wavenumbers is highly corelated we expect this 'regional' behavior for both sign and magnitude of model coefficients, reflecting that subsequent data-points has similar effect on the DP3+ GOS prediction.

**Example 13**

Reconstituted milk samples were prepared by diluting 4.1 %, 9.8 %, 20.5 % or 30.76 % skimmed milk powder in tap water. Zymstar GOS (Material A15017, batch 4863445828) was added in a dose of either 0.7 g/L, 1.5 g/L, 3 g/L, 6 g/L, 12 g/L or 18 g/L and then incubated at either 5 °C, 10 °C or 45 °C. After 2, 4, 7, 16 and 24 hours a sample was extracted and incubated at 95 °C for 12 minutes for inactivation of enzyme. The resulting samples were subjected to Fourier Transform infrared (FTIR) Spectroscopy and the individual carbohydrates in the samples were quantified according to example 1 and 2. For samples with 30.75 % skimmed milk powder FTIR measurements were performed both prior to and after they were diluted 1:3 in water.

**Example 14:**

Several other model types than the (PLSR)-model can be used for prediction of the concentration of GOS in milk samples derived from Milkoscan data-files. Given enough data, one obvious choice is a Neural Network. The FTIR spectra (or alternatively some specific spectra region) can be used as input data, with the DP3+ GOS concentrations as measured by HPLC as target values and DP3+ GOS predictions as output of the Neural Network. A Neural Network is comprised of a set of units called nodes. Typically, these nodes are arranged in layers, with connections from one layer to the next, such that signals travel from the input layer (the FTIR spectrum) to the output layer (DP3+ GOS prediction), possibly having multiple layers in between. Each node takes a weighted sum of incoming connections as input and computes an output based on a nonlinear activation function. The algorithm is optimized by adjusting the weights, typically to minimize the mean squared error between target and output. Often regularization is also implemented to prevent overfitting. One example is L2 regularization, where the loss function is penalized with the sum of squared weights times a regularization parameter $\alpha$. Neural Networks comes in many forms and structures, an overview can be found in (Schmidhuber, 2015). Often some structures of the Neural Network are better suited for a specific class of problems than others. Multiple examples of using Neural Nets within chemometric can be found in the literature, amongst others (Cui & Fearn, 2018).

Python offers several frameworks for implementation of Neural Networks, hereunder sklearn.neural_network and tensorflow.kereas. Generally, many hyperparameters can be adjusted to find the best model for the problem at hand. For the following example scikitlearn v. 0.22.1 was used.

A Neural Network was trained and evaluated on a dataset comprising of 244 fresh milk samples and 377 reconstituted milk samples prepared similar to example 4-8 and example 13 using milk bases with an initial lactose content ranging from 2% to 15%. 80% of the dataset was used for training and 20% for evaluating the model. Only data within the spectral range 1041 cm$^{-1}$-1450 cm$^{-1}$ was used, and it was preprocessed by applying sklearn.preprocesseing.Normaliser (normalizing each sample to unit norm) and subsequent detrending. The python package sklearn.neural_network.MLPRegressor was used to create the Neural Network, and a cross validation grid search was performed, dividing into 5 cross validation data-sets and using the set of hyperparameters listed in table 8. For specification of the model and meaning of each hyperparameter see Scikit-learn: Machine Learning in Python, Pedregosa et al., JMLR 12, pp. 2825-2830, 2011 version 0.22.1. In table 8 the notation indicating number and size of the layers uses soft brackets (). The number of integers within the brackets, indicate the number of hidden layers, while the integers themselves indicate the number of nodes in each of these layers. For all runs the maximal number of iterations were set to 4000, all other parameters were set as defaults of ref. 5. The best combination of hyperparameters from the available parameters stated in table 8 were found to be {Layer size = (106) ; Optimization algorithm = LBFGS ; $\alpha$ = 5*10$^{-5}$; Activation Function = Tanh function}. Results of this model are reported as **model#9** in table 9, reflecting a model performance for the DP3+ GOS-prediction of a R-squared $R^2$ = 0.961 and a root-mean-squared error of prediction RMSEP = 0.29. If comparing to **model#6,** keep in mind that **model#6** only contains fresh milk samples, while **model#9** also includes reconstituted milk samples, while having almost as good an R-squared.

The raw FTIR spectra of reconstituted milk having a high number of solids were found to differ more from the fresh-milk samples of the dataset than reconstituted milk having a lower number of solids. And since one may not want to use all FTIR instruments on samples with high solids, it was investigated if the model had been compromised by including diluted samples. Where **model#9** included all samples from example 13 the diluted samples were excluded from **model#10.** The hyperparameters were set to match the hyperparameters of **model#9,** i.e. they were set to {Layer size = (106) ; Optimization algorithm = LBFGS ; $\alpha$ = 5*10$^{-5}$ ; Activation Function = Tanh function}. Results of this model are reported as **model#10** in table 9, with a R-squared $R^2$ = 0.962 and a root-mean-squared error of prediction RMSEP = 0.30. I.e. there is almost no difference in model performance compared to **model#9.**

Similar methodology was used to predict the glucose, galactose, DP2 and lactose as measured by example 1 and 2. With the prediction of all the carbohydrates as measured in example 1 and 2 one would be able to apply example 3 to predict the total GOS concentration.

Testing a similar Neural Network on the entire wavenumber range 926:5010 cm$^{-1}$, gives slightly lower performance than using the wavenumber region 1041:1450 cm$^{-1}$. The test was performed using cross validation grid search over hyperparameters listed in table 8 with an additional layer size option of (1029), reflecting that the input now contains 1030 data-points. The optimal combination of hyperparameters were found to be {Layer size = (150) ; Optimization algorithm =LBFGS ; $\alpha$ = 5*10$^{-4}$ ; Activation Function = Identity}. Resulting in an R-squared $R^2$ = 0.928 and a root-mean-squared error of prediction RMSEP = 0.39, see **model#11** in table 9.

Table 8:

| Layer size | (1) ; (8) ; (50) ; (100) ; (106) ; (150) ; (8,8) ; (50,25) ; (100,50 ) |
|---|---|
| Optimization algorithm | LBFGS ; SGD; Adam ; |
| $\alpha$ (L2 penalty parameter) | 5*10$^{-5}$ ; 5*10$^{-4}$ |
| Activation Function | Thanh function ; Logistic Sigmoid Function ; Rectified linear units ; Identity |

Table 9:

| Model number | Spectral range in wavenumbers cm$^{-1}$ | $R^2$ of Neural Network regression model – should be close to 1.0 | Error (prediction) of Neural Network-regression model (RMSE) – should be as low as possible | Comments |
|---|---|---|---|---|
| Model#9 | 1041:1450 | 0.961 | 0.288 | Range used for the DP3+ model |
| Model #10 | 1041:1450 | 0.962 | 0.298 | Without diluted samples |
| Model #11 | 926:5010 | 0.928 | 0.392 | Full range |

References:

Cui, C., & Fearn, T. (2018). Modern practical convolutional neural networks for multivariate regression: Applications to NIR calibration. Chemometrics and Intelligent Laboratory Systems, 182, 9-20.

Grelet, C., Fernández Pierna, J. A., Dardenne, P., Baeten, V., & Dehareng, F. (2015). Standardization of milk mid-infrared spectra from a European dairy network. Journal of Dairy Science, 98, 2150-2160.

Martens, H., Izquierdo, L., Thomassen, M., & Martens, M. (1986). Partial least-squares regression on design variables as an alternative to analysis of variance. Analytica Chimica Acta, 191, 133-148.

Schmidhuber, J. (2015). Deep learning in neural networks: An overview. Neural Networks, 61, 85-117.

Ref. 5. See SK Learn Neural network at the website: scikit-learn.org/stable/modules/generated/sklearn.neural_network.MLPRegressor.html

**Claims**

1. A method for determining carbohydrate content in a sample, the method comprising:

   obtaining FTIR (Fourier Transform Infrared Spectroscopy) spectrum data corresponding to the sample;
   providing at least a portion of the FTIR spectrum data as an input to a trained machine learning model; and
   processing at least a portion of the FTIR spectrum data using the trained machine learning model to generate a carbohydrate content value providing a quantitative indication of a level of carbohydrate content in the sample.

2. The method according to claim 1 wherein the sample is a milk-based substrate.

3. The method according to any of the previous claims, in which the portion of the FTIR spectrum data supplied as the input to the trained machine learning model comprises FTIR spectrum data within a limited spectral range, wherein preferably the limited spectral range includes 1046cm$^{-1}$, 1076cm$^{-1}$, 1157 cm$^{-1}$ and 1250 cm$^{-1}$.

4. The method of claim 3, in which the limited spectral range comprises a wavenumber region for which a lower bound is between 900 cm$^{-1}$ and 1100 cm$^{-1}$ and an upper bound is between 1300 cm$^{-1}$ and 1500 cm$^{-1}$, wherein preferably the lower bound is between 1008 cm$^{-1}$ and 1068 cm$^{-1}$ and the upper bound is between 1414 cm$^{-1}$ and 1475 cm$^{-1}$, wherein more preferably the limited spectral range comprises wavenumber region 1037:1450 cm$^{-1}$.

5. The method according to any of the preceding claims, in which the trained machine learning model comprises a supervised learning model trained using a training data set comprising, for each of a plurality of training samples, the FTIR spectrum data corresponding to the training sample and a measured indication of the level of carbohydrate content in the training sample.

6. The method according to any of the preceding claims, in which the trained machine learning model comprises:

    (a) a partial least squares regression (PLSR) model;
    (b) a Neural Network regression model;
    (c) multiple-linear regression (MLR);
    (d) principle components regression (PCR); or
    (e) classical least squares method (CLS).

7. The method according to any of the previous claims, in which the FTIR spectrum data is obtained from a server-based data store to which the FTIR spectrum data is uploaded by a client device, wherein preferably the carbohydrate content value is made accessible to the client device.

8. The method according to any of the previous claims, in which the processing of at least a portion of the FTIR spectrum data using the trained machine learning model is performed at a server device using the FTIR spectrum data obtained from a client device, wherein preferably the carbohydrate content value is made accessible to the client device.

9. A method for training a machine learning model to predict carbohydrate content in a milk-based substrate; the method comprising:

    obtaining a training data set comprising, for each of a plurality of training samples, FTIR (Fourier Transform Infrared Spectroscopy) spectrum data corresponding to the training sample and a measured indication of a level of carbohydrate content in the training sample; and
    performing supervised learning using the training data set, to determine trained model coefficients for the machine learning model.

10. A computer program which, when executed on a data processing apparatus, controls the data processing apparatus to perform the method of any of the previous claims.

11. A method for preparing a milk product containing carbohydrate, comprising:

    treating a milk-based substrate with a trans-galactosylating enzyme;
    performing FTIR (Fourier Transform Infrared Spectroscopy) on a sample of the milk-based substrate to obtain FTIR spectrum data corresponding to the sample;
    obtaining, based on processing of at least a portion of the FTIR spectrum data using a trained machine learning model, a carbohydrate content value providing a quantitative indication of a level of carbohydrate content in the sample; and
    determining, based on the carbohydrate content value, when to inactivate the trans-galactosylating enzyme by pasteurization of the milk base.

12. The method of claim 11, in which at least a portion of the FTIR spectrum data is uploaded by a client device to a server for server-based processing by the trained machine learning model to generate the carbohydrate content value, and the carbohydrate content value is obtained by the client device as a result of the server-based processing.

13. The method of claim 11 or claim 12 having an accuracy better than 10%, expressed as Standard Error of Prediction at mean value (3.75 %), the concentration of GOS in a concentration range of 0-7.5 % in a milk base containing at least 0.1% fat, at least 0.5% dissolved lactose, and at least 1% protein.

14. The method of any of claims 11 to 13 having a linearity ($R^2$) of the PLS regression model above 0.9 to validate the GOS content.

15. The method according to any of the previous claims wherein the carbohydrate is one or more of GOS, DP3+ GOS, glucose, galactose, and DP2, wherein preferably DP2 is lactose and/or the carbohydrate is DP3+ GOS.

16. The method of any of claims 11 to 15 wherein the trans-galactosylating enzyme is derived from *Bifidobacterium bifidum,* wherein preferably the enzyme is a truncated β-galactosidase from *Bifidobacterium bifidum,* most preferably truncated on the C-terminus.

17. The method of claim 16 wherein the truncated β-galactosidase from *Bifidobacterium bifidum* comprises a polypeptide having at least 70%, at least 80%, at least 90%, at least 95% or at least 99% sequence identity to SEQ ID. NO:1, SEQ ID NO:2, SEQ ID NO:3, SEQ ID NO:4 or SEQ ID NO:5 or to a transgalactosylase active fragment thereof, wherein preferably the polypeptide comprises a sequence according to SEQ ID. NO:1, SEQ ID NO:2, SEQ ID NO:3, SEQ ID NO:4 or SEQ ID NO:5 or a transgalactosylase active fragment thereof, wherein most preferably the polypeptide comprises a sequence according to SEQ ID. NO:1.

FIG. 1

FIG. 2

FIG. 3

FIG. 4

FIG. 5

FIG. 6

FIG. 7

Europäisches
Patentamt
European
Patent Office
Office européen
des brevets

# EUROPEAN SEARCH REPORT

Application Number

EP 21 16 7494

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PICQUE D ET AL: "Monitoring of fermentation by infrared spectrometry", ANALYTICA CHIMICA ACTA, ELSEVIER, AMSTERDAM, NL, vol. 279, no. 1, 1 July 1993 (1993-07-01), pages 67-72, XP026594669, ISSN: 0003-2670, DOI: 10.1016/0003-2670(93)85067-T [retrieved on 1993-07-01] | 1,3-10 | INV. G16B40/20 |
| Y | * whole document, in particular abstract, Materials and Methods and Discussiona an perspectives * | 2,11-17 | |
| X | RODRIGUEZ-SAONA L E ET AL: "Rapid analysis of sugars in fruit juices by FT-NIR spectroscopy", CARBOHYDRATE RESEARCH, PERGAMON, GB, vol. 336, no. 1, 1 November 2001 (2001-11-01), pages 63-74, XP004310468, ISSN: 0008-6215, DOI: 10.1016/S0008-6215(01)00244-0 | 1,5-10 | |
| | | | TECHNICAL FIELDS SEARCHED (IPC) |
| Y | * whole document, in particular abstract; Table 1 and Figures 3-4; Materials and Methods * | 2,11-17 | G16B |
| | -/-- | | |

The present search report has been drawn up for all claims

| Place of search | Date of completion of the search | Examiner |
|---|---|---|
| Munich | 15 September 2021 | Vanmontfort, D |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 1 of 3

Europäisches
Patentamt

European
Patent Office

Office européen
des brevets

# EUROPEAN SEARCH REPORT

Application Number

EP 21 16 7494

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DIAS L G ET AL: "UV spectrophotometry method for the monitoring of galacto-oligosaccharides production", FOOD CHEMISTRY, ELSEVIER LTD, NL, vol. 113, no. 1, 1 March 2009 (2009-03-01), pages 246-252, XP025474670, ISSN: 0308-8146, DOI: 10.1016/J.FOODCHEM.2008.06.072 [retrieved on 2008-07-05] * whole document, in particular abstract; page 246, column 2, last paragraph; Experimental on pages 247-248; Figures 1 and 3; Tables; Conclusions * | 11-17 | |
| A | ERDOS BALÁZS ET AL: "Artificial Neural Network-Assisted Spectrophotometric Method for Monitoring Fructo-oligosaccharides Production", FOOD AND BIOPROCESS TECHNOLOGY ; AN INTERNATIONAL JOURNAL, SPRINGER-VERLAG, NEW YORK, vol. 11, no. 2, 25 October 2017 (2017-10-25), pages 305-313, XP036410044, ISSN: 1935-5130, DOI: 10.1007/S11947-017-2011-3 [retrieved on 2017-10-25] * Whole document, in particular abstract, page 308 of Materials and methods, Result and Discussion and Conclusions * | 1-17 | |

TECHNICAL FIELDS SEARCHED (IPC)

-/--

The present search report has been drawn up for all claims

| Place of search | Date of completion of the search | Examiner |
|---|---|---|
| Munich | 15 September 2021 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 16 7494

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NICOLAOU N. ET AL: "Fourier transform infrared spectroscopy and multivariate analysis for the detection and quantification of different milk species", JOURNAL OF DAIRY SCIENCE, vol. 93, no. 12, 1 December 2010 (2010-12-01), pages 5651-5660, XP055840500, US ISSN: 0022-0302, DOI: 10.3168/jds.2010-3619 * whole document, in particular abstract; Materials and Methods; Conclusions; Figure 1 and Table 1 * | 2,11-17 | |

TECHNICAL FIELDS
SEARCHED     (IPC)

The present search report has been drawn up for all claims

| Place of search | Date of completion of the search | Examiner |
|---|---|---|
| Munich | 15 September 2021 | Vanmontfort, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## REFERENCES CITED IN THE DESCRIPTION

### Patent documents cited in the description

- WO 2013182686 A
- WO 2020117548 A
- WO 9117243 A
- EP 238023 A
- US 6022725 A

### Non-patent literature cited in the description

- **SAMBROOK et al.** Molecular Cloning: A Laboratory Manual. 1989
- Oligonucleotide Synthesis. 1984
- Current Protocols in Molecular Biology. 1994
- PCR: The Polymerase Chain Reaction. 1994
- **KRIEGLER.** Gene Transfer and Expression: A Laboratory Manual. 1990
- The Alcohol Textbook. 2009
- *Essentials of Carbohydrate Chemistry and Biochemistry,* 2007
- **SINGLETON et al.** Dictionary of Microbiology and Molecular Biology. John Wiley and Sons, 1994
- **HALE ; MARKHAM.** The Harper Collins Dictionary of Biology. 1991
- **THOMPSON et al.** *Nucleic Acids Res.,* 1994, vol. 22, 4673-4680
- **LIU et al.** Improved heterologous gene expression in Trichoderma reesei by cellobiohydrolase I gene (cbh1) promoter optimization. *Acta Biochim. Biophys. Sin (Shanghai),* 2008, vol. 40 (2), 158-65
- **SAMBROOK et al.** MOLECULAR CLONING: A LABORATORY MANUAL. Cold Spring Harbor, 1989
- MOLECULAR CLONING: A LABORATORY MANUAL. 2001
- **CAO et al.** *Science,* 2000, vol. 9, 991-1001
- *CHEMICAL ABSTRACTS,* 67-68-5
- *CHEMICAL ABSTRACTS,* 7664-38-2
- *CHEMICAL ABSTRACTS,* 64-19-7
- *CHEMICAL ABSTRACTS,* 7558-79-4
- *CHEMICAL ABSTRACTS,* 150-13-0
- *CHEMICAL ABSTRACTS,* 3999-38-0
- *CHEMICAL ABSTRACTS,* 32503-27-8
- *CHEMICAL ABSTRACTS,* 10039-26-6
- **PEDREGOSA et al.** Scikit-learn: Machine Learning in Python. *JMLR,* 2011, vol. 12, 2825-2830
- **CUI, C. ; FEARN, T.** Modern practical convolutional neural networks for multivariate regression: Applications to NIR calibration. *Chemometrics and Intelligent Laboratory Systems,* 2018, vol. 182, 9-20
- **GRELET, C. ; FERNÁNDEZ PIERNA, J. A. ; DARDENNE, P. ; BAETEN, V. ; DEHARENG, F.** Standardization of milk mid-infrared spectra from a European dairy network. *Journal of Dairy Science,* 2015, vol. 98, 2150-2160
- **MARTENS, H. ; IZQUIERDO, L. ; THOMASSEN, M. ; MARTENS, M.** Partial least-squares regression on design variables as an alternative to analysis of variance. *Analytica Chimica Acta,* 1986, vol. 191, 133-148
- **SCHMIDHUBER, J.** Deep learning in neural networks: An overview. *Neural Networks,* 2015, vol. 61, 85-117